(19) Europäisches Patentamt
European Patent Office
Office européen des brevets

(11) Veröffentlichungsnummer: **0 432 740 A2**

(12) **EUROPÄISCHE PATENTANMELDUNG**

(21) Anmeldenummer: **90123853.5**

(22) Anmeldetag: **11.12.90**

(51) Int. Cl.5: **C07D 277/30**, A61K 31/425, C07D 417/06, C07D 417/12

(30) Priorität: **15.12.89 DE 3941438**

(43) Veröffentlichungstag der Anmeldung: **19.06.91 Patentblatt 91/25**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Thorwart, Werner, Dr.**
**Am Gänsborn 3b**
**W-6203 Hochheim am Main(DE)**
Erfinder: **Schleyerbach, Rudolf, Dr.**
**Finkenweg 10**
**W-6238 Hofheim am Taunus(DE)**
Erfinder: **Bartlett, Robert, Dr.**
**Sandbergstrasse 20**
**W-6100 Darmstadt(DE)**
Erfinder: **Weithmann, Klaus Ulrich, Dr.**
**Am Domherrnwald 18**
**W-6238 Hofheim am Taunus(DE)**

(54) **Neue 2-substituierte 4-(3-Alkyl-5-tert.-butyl-4-hydroxyphenyl)-thiazole, Verfahren zu ihrer Herstellung, die sie enthaltenden Arzneimittel und ihre Verwendung.**

(57) Neue substituierte Thiazole der allgemeinen Formel I, in der

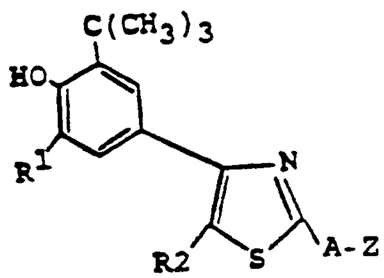

(I)

$R^1$
eine gesättigte oder ungesättigte, geradkettige oder verzweigte $C_1$-$C_5$-Alkylgruppe,
$R^2$
ein Wasserstoffatom oder ein Alkylrest mit 1 bis 3 C-Atomen,
A
eine Zwischenkette der Formel

$-(CH_2)_n-Y-CR^3R^4-$; $-CH=CR^3-(CH_2)_m-$ oder $-CH=N-O-(CH_2)_n-$

wobei Y eine Einfachbindung, ein Sauerstoff- oder Schwefelatom oder eine Carbonylgruppe, $R^3$ und $R^4$ gleich oder verschieden sind und ein Wasserstoffatom oder einen Alkylrest mit bis zu 2 C-Atomen, m eine Zahl von 0 bis 3 und n eine Zahl von 1 bis 4 darstellen, und
Z
eine Tetrazol-, CN-Gruppe oder einen Rest der Formel

EP 0 432 740 A2

O

-C-X

X

eine Hydroxygruppe oder ein Rest der allgemeinen Formel $R^5O$- oder $R^6R^7N$- , wobei $R^5$ ein geradkettiger oder verzweigter, gegebenfalls durch Hydroxy, $C_1$-$C_3$-Alkoxy oder $C_1$-$C_3$-Alkylamino-substituierter $C_1$-$C_4$-Alkylrest, $R^6$ und $R^7$ gleich und verschieden sind und ein Wasserstoffatom; ein geradkettig oder verzweigter $C_1$-$C_6$-Alkylrest oder für den Fall, daß $R^6$ ein Wasserstoffatom oder ein $C_1$-$C_4$-Alkylrest bedeutet, $R^7$ eine Hydroxy-, $C_1$-$C_3$-Alkoxy- oder eine Tetrazol-5-yl-Gruppe oder X mit den Strukturelementen -A-(C=O)-zusammen einen Rest der allgemeinen Formel II darstellt,

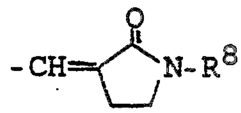

(II)

wobei $R^8$ ein Wasserstoffatom, einen $C_1$-$C_3$-Alkyl- oder $C_1$-$C_3$-Alkoxyrest bedeutet, sowie physiologisch verträgliche Salze solcher Verbindungen der allgemeinen Formel I, in denen X eine Hydroxy- oder Hydroxyaminogruppe darstellt,

werden nach verschiedenen Verfahren hergestellt.

Sie eignen sich bevorzugt zur Behandlung und Prophylaxe von entzündlichen - insbesondere entzündlich rheumatischen - Erkrankungen.

# NEUE 2-SUBSTITUIERTE 4-(3-ALKYL-5-TERT.-BUTYL-4-HYDROXYPHENYL)-THIAZOLE, VERFAHREN ZU IHRER HERSTELLUNG, DIE SIE ENTHALTENDEN ARZNEIMITTEL UND IHRE VERWENDUNG

Die vorliegende Erfindung betrifft neue 2-substituierte 4-(3-Alkyl-5-tert.-butyl-4-hydroxyphenyl)-thiazole, Verfahren zu ihrer Herstellung und ihre Verwendung als Wirkstoffe in Arzneimitteln zur Behandlung entzündlicher, insbesondere entzündlich rheumatischer Erkrankungen.

Es ist bekannt, daß ein genereller Nachteil aller klassichen nicht-steroidalen Antiphlogistika darin besteht, daß sie zwar die Beseitigung oder Linderung der Symptome Schmerz, Entzündung und Schwellung gestatten, aber jene pathologischen Prozesse weitestgehend unbeeinflußt lassen, die den progredienten Verlauf der entzündlichen rheumatischen Erkankungen mitbedingen. Es besteht somit ein dringendes Bedürfnis nach therapeutisch nutzbaren Antirheumatika, die aufgrund ihres Wirkprofils einen tiefgreifenden und nachhaltigen Eingriff in den Entzündungsprozess erwarten lassen. Erfolgversprechende Ansatzpunkte hierzu bieten solche Pharmaka, die neben einer Hemmung der Cyclooxygenase verstärkt in den alternativen Weg des Arachidonsäuremetabolismus eingreifen, (indem sie beispielsweise die 5-Lipoxygenase hemmen und somit die exzessive Bildung der proinflammatorischen Leukotriene unterbinden) und die gegebenenfalls auch antioxidative Eigenschaften besitzen und daher die hochreaktiven Sauerstoffradikale, die als Entzündungsmediatoren in den rheumatischen Gelenken eine fortschreitende Zell- und Gewebszerstörung bewirken, desaktivieren.

Pharmaka mit dieser Indikationsrichtung sind z.B. beschrieben in der EP-A-0276805; es handelt sich um Produkte auf der Basis von substituierten 3-Phenyl-7H-thiazolo-[3,2-b][1,2,4]triazin-7-onen der folgenden allgemeinen Formel

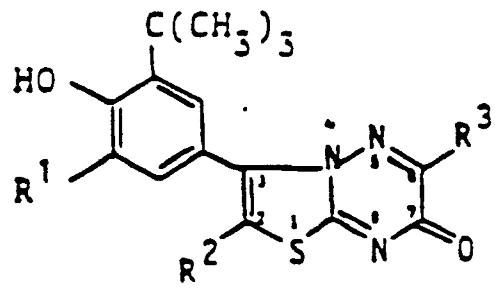

in der

$R^1$

eine geradkettige oder verzweigte Alkylgruppe mit 1 bis 4 C-Atomen, Hydroxymethyl oder eine Aminomethylgruppe der Formel

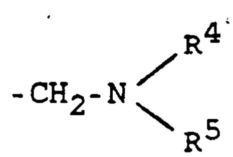

$R^2$

ein Wasserstoffatom oder einen Alkylrest mit 1 bis 3 C-Atomen und

$R^3$

ein Wasserstoffatom, eine geradkettige oder verzweigte Alkylgruppe mit 1 bis 4 C-Atomen, Hydroxymethyl oder die vorerwähnte Aminomethylgruppe bedeuten, wobei

$R^4$ und $R^5$

gleich oder verschieden sind und ein Wasserstoffatom oder einen geradkettigen oder verzweigten Alkylrest mit 1 bis 4 C-Atomen darstellen, oder beide Reste zusammen mit dem Stickstoffatom, an das sie gebunden sind, einen 5- bis 7-gliedrigen gesättigten Ring mit 4 bis 6 C-Atomen oder mit 4 oder 5 C-Atomen und zusätzlich einem weiteren Heteroatom in Form von O, S oder $NR^6$ bilden und $R^6$ die Bedeutung von Wasserstoff oder $(C_1\text{-}C_4)$Alkyl hat.

Weiterhin sind in bestimmter Weise substituierte Thiazol-Derivate bekannt, welche neben einer antiatherosklerotischen Wirkung entzündungs- und fiebermildernde sowie schmerzlindernde Eigenschaften besitzen sollen (DE-A-16 95 252); die pharmakologischen Effekte werden dort aber nicht näher erläutert. Für diese Thiazolderivate sind 2 Substituenten charakteristisch, nämlich

- ein Phenyl- oder Aralkylrest, der im Phenyl- bzw. Aryl-Teil seinerseits durch Halogen, $NO_2$ oder $CF_3$ substituiert sein kann, sowie
- ein Rest der Formel $-CH_2-COOH$ in dieser oder einer davon abgeleiteten Form.

Auch die - mit dem 3,5-Di-tert.-butyl-4-hydroxyphenyl-Rest substituierten - Thiazolderivate der folgenden allgemeinen Formel sollen anti-inflammatorische und schmerzlindernde (analgetische) Eigenschaften besitzen (JP-A-87,132,871):

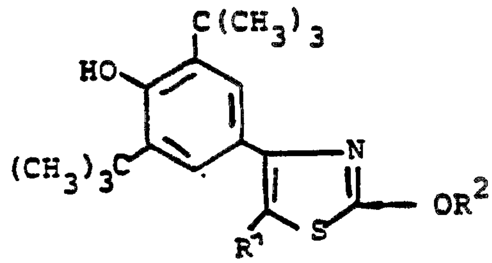

worin

$R^1$ = H,
Hydroxy(nieder)alkyl,
Hydroxyimino(nieder)alkyl,
Niederalkoxyimino(nieder)alkyl,
-CHO, -COOH, -CN, $-NO_2$, Halogen, -NCS oder Alkylenamino, und

$R^2$ = H,
Niederalkyl,
Niederacyl,
Niederalkoxycarbonyl

mit der Maßgabe, daß im Falle
$R^1$ = H $R^2$ = Niederacyl oder Niederalkoxycarbonyl sein muß.

Von ähnlicher Struktur sind auch die in der US-A-4 535 165 u.a. beschriebenen 3,5-Di-tert.-butyl-hydroxyphenyl-thiazol-Derivate der folgenden Formel:

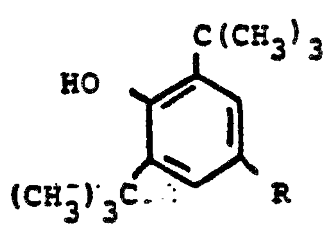

worin

R = Thiazolyl,
Halothiazolyl oder
Methylthiazolyl.

Die Verbindungen sollen anti-inflammatorisch wirksam sein.

In dem Bestreben, das eingangs beschriebene Bedürfnis nach therapeutisch nutzbaren Antirheumatika, die aufgrund ihres Wirkprofils einen tiefgreifenden und nachhaltigen Eingriff in den Entzündungsprozeß erwarten lassen, möglichst besser als bisher zu befriedigen, wurde nun gefunden, daß dieses Ziel durch die Bereitstellung neuer Thiazolderivate erreicht wird, für die speziell ein 3-Alkyl-5-tert.-butyl-4-hydroxyphenyl-Substituent sowie ein weiterer Substituent mit einer Carboxyl- oder einer daraus abgeleiteten Gruppe charakteristisch ist. Infolge der Kombination dieser beiden Substituenten am Thiazolring werden verstärkt die - der chronischen Entzündungsphase zugrundeliegenden - immunpathologischen Prozesse gehemmt und somit ein vorteilhafter antirheumatischer Wirkmechanismus erzielt. Aufgrund ihrer Cyclo- und

Lipoxygenase-hemmenden Eigenschaften, ihrer Fähigkeit, durch ihr antioxidatives Potential Sauerstoffradikale zu desaktivieren sowie ihrer Eigenschaft, vorteilhaft in das gestörte Immunsystem einzugreifen, eignen sich die neuen Thiazolderivate zur Verwendung in Arzneimitteln, insbesondere in solchen, die bei entzündlich rheumatischen Erkrankungen indiziert sind.

Gegenstand der Erfindung sind somit substituierte Thiazole der allgemeinen Formel I,

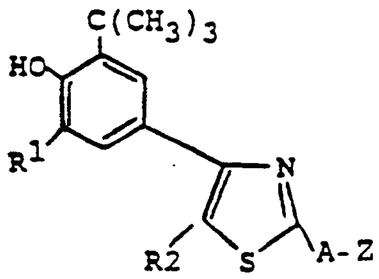

(I),

in der

$R^1$

eine gesättigte oder ungesättigte, geradkettige oder verzweigte $C_1$-$C_5$-Alkylgruppe,

$R^2$

ein Wasserstoffatom oder ein Alkylrest mit 1 bis 3 C-Atomen,

A

eine Zwischenkette der Formel

$-(CH_2)_n-Y-CR^3R^4-$; $-CH=CR^3-(CH_2)_m-$ oder $-CH=N-O-(CH_2)_n-$

wobei Y eine Einfachbindung, ein Sauerstoff- oder Schwefelatom oder eine Carbonylgruppe,

$R^3$ und $R^4$ gleich oder verschieden sind und ein Wasserstoffatom oder einen Alkylrest mit bis zu 2 C-Atomen, und

m eine Zahl von 0 - 3 und

n eine Zahl von 1 bis 4 darstellen, und

Z

eine Tetrazol-, CN-Gruppe oder einen Rest der Formel

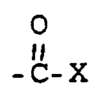

X

eine Hydroxygruppe oder ein Rest der allgemeinen Formel $R^5O$- oder $R^6R^7N$- , wobei $R^5$ ein geradkettiger oder verzweigter, gegebenfalls duch Hydroxy, $C_1$-$C_3$-Alkoxy oder $C_1$-$C_3$-Alkylamino-substituierter $C_1$-$C_4$-Alkylrest, $R^6$ und $R^7$ gleich oder verschieden sind und ein Wasserstoffatom, ein geradkettiger oder verzweigter $C_1$-$C_6$-Alkylrest oder für den Fall, daß $R^6$ ein Wasserstoffatom oder ein $C_1$-$C_4$-Alkylrest bedeutet, $R^7$ eine Hydroxy-, $C_1$-$C_3$- Alkoxy- oder eine Tetrazol-5-yl-Gruppe oder X mit den Strukturelementen -A-(C=O)-zusammen einen Rest der allgemeinen Formel II darstellt,

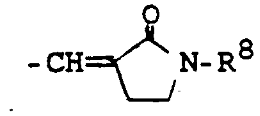

(II)

wobei $R^8$ ein Wasserstoffatom, einen $C_1$-$C_3$-Alkyl- oder $C_1$-$C_3$-Alkoxyrest bedeutet, sowie physiologisch verträgliche Salze solcher Verbindungen der allgemeinen Formel I, in denen X eine Hydroxy- oder Hydroxyaminogruppe darstellt.

Bevorzugt sind dabei solche Verbindungen der Formel I, in denen

$R^1$

eine tert.Butyl- oder Methylgruppe darstellt,

$R^2$

für Wasserstoff oder Methyl,

A

für eine Zwischenkette der Formel

$-(CH_2)_n-Y-CR^3R^4-$

steht, wobei Y eine Einfachbindung oder ein Sauerstoffatom, $R^3$ und $R^4$ gleich oder verschieden sind und ein Wasserstoffatom oder einen Methylrest, und n eine Zahl von 1 oder 2 darstellen, und

X

eine Hydroxygruppe oder ein Rest der allgemeinen Formel $R^5O$- oder $R^6R^7N$- darstellt, wobei $R^5$ ein geradkettiger oder verzweigter $C_1$-$C_3$- Alkylrest, $R^6$ ein Wasserstoffatom oder ein Methylrest und $R^7$ eine Hydroxy-, Methoxy- oder Tetrazol-5-yl-Gruppe bedeutet.

Unter diesen sind wiederum solche Verbindungen der Formel I besonders bevorzugt, in denen

$R^1$

für eine tert.-Butylgruppe

$R^2$

für Wasserstoff oder Methyl,

A

für eine Zwischenkette der Formel

$-(CH_2)_n-Y-CR^3R^4-$

steht, wobei Y eine Einfachbindung, $R^3$ und $R^4$ ein Wasserstoffatom und n eine Zahl von 1 oder 2 darstellen und

X

eine Hydroxygruppe oder einen $R^6R^7N$-Rest darstellt, wobei $R^6$ ein Wasserstoffatom oder ein Methylrest und $R^7$ eine Hydroxy- oder Methoxygruppe bedeutet.

Ganz besonders bevorzugte Verbindung ist 3-[4-(3,5-Di-tert.-butyl-4-hydroxyphenyl)-thiazol-2-yl]-propionsäure, d.i. die Verbindung der Formel I mit

$R^1$ = tert.-Butyl,

$R^2$ = H,

A = $-(CH_2)_n-Y-CR^3R^4-$, worin $R^3 = R^4 = H$, Y = Einfachbindung und n = 1.

Gegenstand der Erfindung sind weiterhin Verfahren zur Herstellung der erfindungsgemäßen Verbindungen der Formel I. Verbindungen der Formel I, in der A eine Zwischenkette der Formel $-(CH_2)_n-Y-CR^3R^4-$, wobei Y, $R^3$, $R^4$ und n die oben angegebene Bedeutung haben und X ein $R^5O$-Rest ist, lassen sich nach Verfahrensweise a) erhalten.

Verfahren a) ist dadurch gekennzeichnet, daß man ein Thiacarbonsäureamid der allgemeinen Formel III

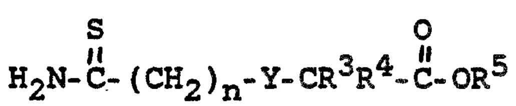

(III)

mit 2-Halogen-1-phenylalkanonen der Formel IV,

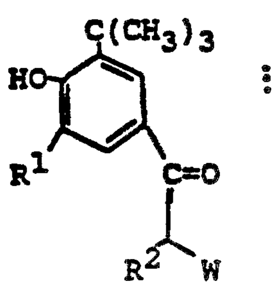

(IV)

in der $R^1$ und $R^2$ die oben genannte Bedeutung haben und W für ein Halogen, vorzugsweise Chlor oder Brom steht, zu den erfindungsgemäßen Verbindungen der Formel Ia,

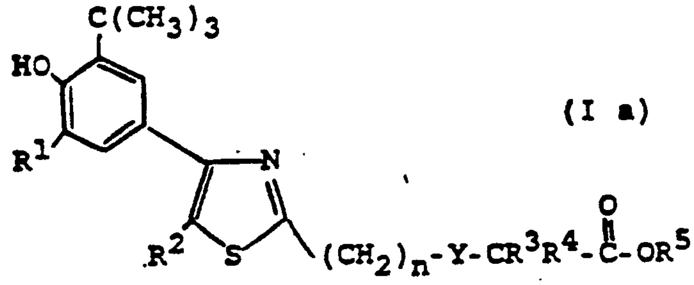

(I a)

mit den für $R^1$, $R^2$, $R^3$, $R^4$, $R^5$, Y und n oben angegebenen Bedeutungen, umsetzt.

Für die Umsetzung, in die man gewöhnlich äquimolare Mengen der Reaktionspartner einsetzt, kommen vor allem polare Solventien in Frage, so beispielsweise Alkohole, wie Methanol, Ethanol, die verschiedenen Propanole oder Butanole aber auch niedere aliphatische Carbonsäuren, wie Ameisensäure und Essigsäure, sowie Essigsäureester, Aceton, Butan-2-on, Dimethylformamid oder Acetonitril oder Mischungen der genannten Lösungsmittel.

Die Reaktion wird im allgemeinen bei Temperaturen zwischen etwa 20 °C und dem Siedepunkt des verwendeten Reaktionsmediums durchgeführt, besonders zwischen etwa 50 °C und 80 °C, wobei die Reaktionszeiten zwischen weniger als einer Stunde und etwa 3 Stunden liegen.

Die für das Verfahren a) notwendigen Thiacarbonamide der allgemeinen Formel III lassen sich nach dem Fachmann bekannten Verfahren herstellen, z. B. durch Addition von Schwefelwasserstoff an die entsprechenden Nitrile in Gegenwart einer Base (s. Houben-Weyl, Methoden der organischen Chemie, Band IX, S. 762 - 768). Bevorzugte Basen, die in katalytischen bis zu äquimolaren Mengen verwendet werden, sind Amine wie beispielsweise Triethylamin, Pyridin aber auch Alkoholate und Alkalihydrogensulfide. Die Reaktion wird in einem organischen Lösungsmittel, insbesondere einem Alkohol wie Methanol, Ethanol oder Propanolen oder in Pyridin durchgeführt; besonders geeignet ist ein Gemisch von Pyridin und Triethylamin. Die Reaktionstemperatur liegt zwischen etwa 0 °C und dem Siedepunkt des verwendeten Lösungsmittels, bevorzugt wird die Reaktion bei Raumtemperatur (etwa 20 - 30 °C) durchgeführt. Eine weitere Ausführungsform des Verfahrens besteht in dem Einsatz von Dithiophosphorsäure-0,0-dialkylestern, insbesondere des Methyl- oder Ethylesters als Schwefelwasserstoffquelle (S. W. Walter und K. D. Bode, Angew. Chem. 1966, 78, S. 517 - 532), bei dem zur Spaltung des primären Anlagerungsproduktes Chlorwasserstoff bei Temperaturen von etwa -10 °C bis 20 °C, bevorzugt etwa -10 °C bis 0 °C, in das Reaktionsgemisch eingeleitet wird.

Die ebenfalls als Ausgangsstoffe verwendeten 2-Halogen-1-phenylalkanone der Formel IV sind literaturbekannt oder lassen sich aus den 1-(3-Alkyl-5-tert.-butyl-4-hydroxyphenyl)-alkanonen durch Umsetzung mit einem geeigneten Halogenierungsmittel nach den im Houben-Weyl Bd. V 4 S. 171 - 189 (1960) beschriebenen Methoden leicht herstellen.

Als geeignete Verbindung IV seien beispielsweise das 2-Brom-1-(3,5-di-tert.-butyl-4-hydroxyphenyl)-ethanon und 2-Brom-1-(3-methyl-5-tert.-butyl-4-hydroxyphenyl)-ethanon genannt, die sich durch Halogenierung der entsprechend substituierten 1-Phenylalkanone mit elementarem Brom oder mit Kupfer-II-bromid nach einem Verfahren von L. C. King und G. K. Ostrum, J. Org. Chem. 1964, 29, S. 3459 - 3461 herstellen lassen.

Zur Gewinnung jener Verbindungen der Formel IV, in denen für Z ein Chloratom steht, eignet sich insbesondere Sulfurylchlorid, das vorzugsweise bei Temperaturen zwischen etwa 10 °C und 30 °C in Anwesenheit inerter Lösungsmittel wie z. B. Methylenchlorid oder Chloroform, mit den entsprechenden 1-Phenylalkanonen zur Reaktion gebracht wird. Eine weiteres Herstellungsverfahren besteht in der Friedel-Crafts-Acylierung von 2-Alkyl-6-tert.-butylphenolen mit vorzugsweise Chloracetylchlorid in Gegenwart von Lewissäuren, wie z. B. Aluminiumchlorid oder Bortrifluorid.

Verbindungen der allgemeinen Formel 1, in denen A = $-CH=CR^3-(CH_2)_m-$ und X vorzugsweise ein $R^5O$-Rest ist, lassen sich nach Verfahren b) herstellen. Verfahren b) ist dadurch charakterisiert, daß man einen Thiazolaldehyd der allgemeinen Formel V,

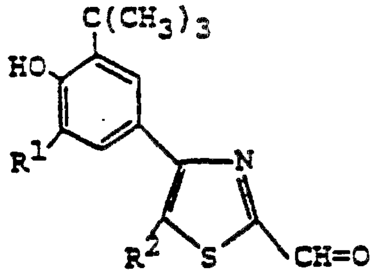

(V)

in einer Olefinierungsreaktion mit einem Phosphonsäuredialkylester der allgemeinen Formel VI

$$(R^9O)_2\overset{O}{\overset{\|}{P}}-CR^3H-(CH_2)_m-Z \qquad (VI)$$

in dem $R^9$ ein $C_1$-$C_3$-Alkylrest ist, zu den erfindungsgemäßen Alkensäureestern der allgemeinen Formel I b,

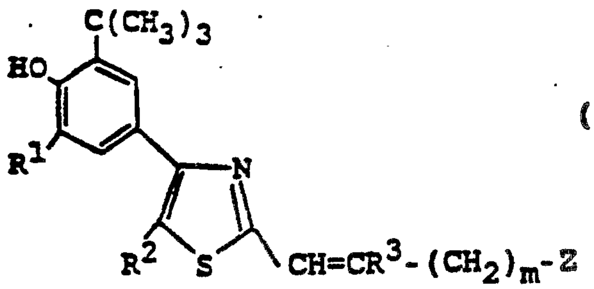

(I b)

in denen $R^1$, $R^2$, $R^3$ und $R^5$ die oben erwähnte Bedeutung haben, umsetzt.

Die hierfür als Zwischenprodukte notwendigen Thiazolaldehyde der allgemeinen Formel V lassen sich vorteilhafterweise durch Umsetzung eines 2-Halogen-1-phenylalkanons der Formel IV mit Dialkoxythioacetamiden, wie beispielsweise Diethoxythioacetamid (vergl. K. Inami und T. Shiba, Bull. Chem Soc. Jap. 1985, 58, S. 352 - 360) gemäß Verfahrensweise a) herstellen. Zur Freisetzung des Aldehyds wird das entsprechende Acetal mit verdünnten Mineralsäuren wie Salz- oder Schwefelsäure erwärmt.

Bevorzugtes Verfahren zur Herstellung der Verbindung I b ist ihre Umsetzung in einer PO-aktivierten Olefinierung mit Phosphorsäuredialkylester der Formel VI, in der $R^3$ ein Wasserstoffatom oder Methyl und $R^5$ und $R^9$ einen Ethylrest bedeuten, unter den dem Fachmann bekannten Standardbedingungen. In einer bevorzugten Ausführungsform wird als Lösungsmittel Dimethylformamid und als Base Natriumhydrid verwendet, wobei die Reaktion im allgemeinen beim Siedepunkt des Lösungsmittel durchgeführt wird.

Verbindungen der allgemeinen Formel I, in denen das Strukturelement -A- zusammen mit -C(=O)X einen Pyrrolidinonrest der Formel II darstellen, lassen sich nach Verfahrensweise c) herstellen. Verfahren c) ist dadurch charakterisiert, daß man einen Thiazolaldehyd der Formel V in einer Olefinierungsreaktion mit einem 3-Halogenpyrrolidinon der Formel VI

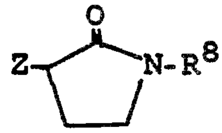

(VII)

I gemeinsam mit einem Phosphin- oder Phosphorsäureester in Gegenwart einer starken Base zu den erfindungsgemäßen Verbindungen I c,

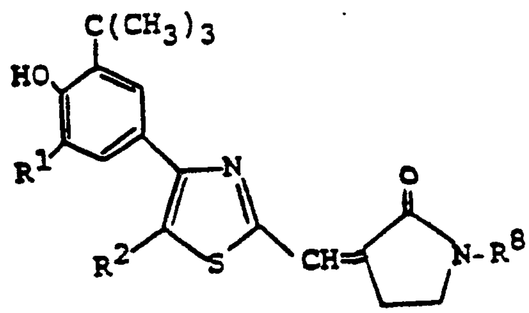

(I c)

mit den für $R^1$, $R^2$, $R^8$ und Z oben angeführten Bedeutungen, umsetzt.

Die bevorzugte Ausführungsform des Verfahrens c) ist die Umsetzung eines 3-Halogenpyrrolidinons der Formel VII, insbesondere solcher, in denen Z ein Bromatom ist, mit einem Phosphin wie Triphenylphosphin zu den entsprechenden quartären Phosphoniumsalzen, wobei im Falle von $R^8$ = Wasserstoff zum Schutze des freien Amidstickstoffes zuvor Verbindung VII mit einem Acetylierungsmittel wie Acetanhydrid behandelt wird. Diese Zwischenstufen lassen sich im allgemeinen ohne weitere Aufarbeitung nach den für eine Wittig-Reaktion üblichen Standardverfahren mit den Aldhyden der Formel V umsetzen. Besonders vorteilhaft erwies sich dabei das Erwärmen der Reaktionspartner in einem inerten Lösungsmittel, insbesondere einem Alkohol wie Ethanol oder Dimethylformamid in Gegenwart einer Base wie Triethylamin, einem Natrium- oder Kaliumalkoholat, Natrium- oder Kaliumhydrid oder Natrium- bzw. Kaliumhydroxid. Die Reaktion wird bei Temperaturen von etwa 20 °C bis zum Siedepunkt des verwendeten Lösungsmittels, bevorzugt in Bereich von etwa 60 ° - 80 °C durchgeführt.

Verbindungen der allgemeinen Formel I, in denen A ein Oxim der Formel $-CH=N-O-(CH_2)_n-$ als Zwischenkette beinhaltet, lassen sich nach Verfahren d) herstellen. Verfahren d) zeichnet sich dadurch aus, daß man eine Verbindung der Formel V mit einem Hydroxylamin der Formel VIII, in dem

$$H_2N\text{-}O\text{-}(CH_2)_n\text{-}\overset{O}{\overset{\|}{C}}\text{-}X \qquad \text{(VIII)}$$

X und n die oben genannte Bedeutung haben, zu den erfindungsgemäßen Verbindungen I d

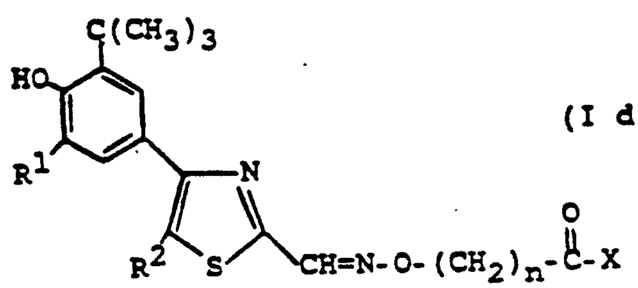

(I d)

kondensiert.

Die Umsetzung erfolgt vorteilhafterweise mit äquimolaren Mengen der Reaktionspartner in wäßrig-alkoholischer Lösung; aber auch in anderen inerten Lösungsmitteln wie Pyridin, Dimethylformamid und Alkoholen wie Methanol, Ethanol und die verschiedenen Propanole und Butanole sowie in Gemischen dieser Lösungsmittel. Die Hydroxylaminderivate der Formel VIII werden dabei zweckmäßig in Form ihrer Säureadditionssalze, wie der Hydrochloride, Hydrobromide oder Sulfate eingesetzt. In diesem Falle empfiehlt es sich, ein säurebindendes Mittel, beispielsweise Alkalihydroxide oder -carbonate oder auch eine organische Base, wie Triethylamin in mindestens stöchiometrischer Menge zuzusetzen. Die Reaktion wird bei Temperaturen zwischen etwa 20 °C und dem Siedepunkt des Lösungsmittels, vorzugsweise zwischen 40 °C und 70 °C durchgeführt.

Verbindungen der allgemeinen Formel I, in denen in der Zwischenkette A für Y ein Sauerstoffatom, $R^3$ und $R^4$ ein $C_1$-$C_2$-Alkylrest, n = 1 und für X eine Hydroxygruppe steht, lassen sich auch nach Verfahren e)

herstellen. Verfahren e) ist dadurch charakterisiert, daß man ein Hydroxymethylthiazol der Formel IX,

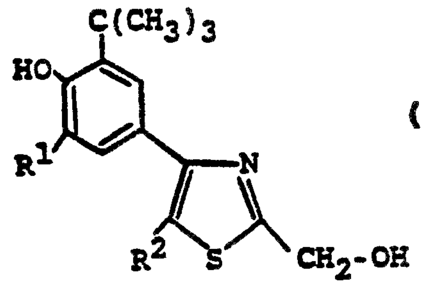

(IX)

mit einem Keton der Formel $R^3$-CO-$R^4$, wobei $R^1$, $R^2$ und $R^4$ die oben angegebene Bedeutung haben, in Gegenwart eines tri- oder tetrahalogenierten Alkans und einer starken Base in die erfindungsgemäßen Verbindungen der Formel I e,

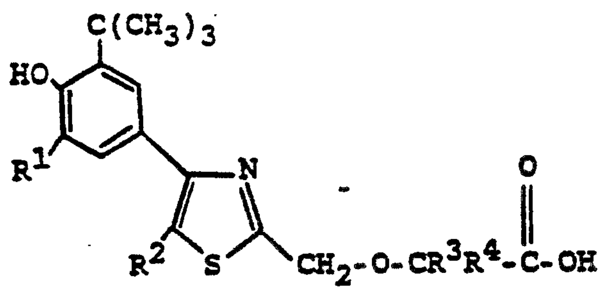

(I e)

umsetzt.

Die dazu erforderlichen Hydroxymethylthiazole der allgemeinen Formel IX lassen sich durch Reduktion der entsprechenden Aldehyde der Formel V nach, im Prinzip bekannten Methoden herstellen. Als Reduktionsmittel eignen sich komplexe Hydride wie Natrium- oder Lithiumborhydrid oder Lithiumaluminiumhydrid. Die Umsetzung erfolgt bevorzugt in einem inerten organischen Lösungsmittel, wie Tetrahydrofuran, einem Ether wie beispielsweise Diethyl- oder tert.-Butylmethylether, Toluol oder Methylenchlorid in einem bevorzugtem Temperaturbereich von etwa 20° C bis 80° C.

Ein weiteres Verfahren zur Herstellung der Vorstufen der allgemeinen Formel IX besteht gemäß Verfahrensvariante a) in der Reaktion eines 2-Halogen-1-phenylalkanons der Formel IV mit Benzoyloxythioacetamid (J. F. Olin und T. B. Johnson, Recl. Trav. Chim. Pays-Bas 1931, 50, 72) sowie einer nachfolgenden Hydrolyse der Benzoyl-Schutzgruppe in Gegenwart einer Base wie Natrium- oder Kaliumhydroxid oder -alkoholat in Alkoholen, wie Methanol oder Ethanol als bevorzugtes Lösungsmittel.

Zur Darstellung der Verbindungen der Formel I e werden Alkohole der Formel IX mit tri- oder tetrasubsituierten Alkanen, wie Bromoform, Jodoform, Tetrachlorkohlenstoff und insbesondere Chloroform mit einem Keton wie beispielsweise Aceton, Butanon-2 oder Pentanonen unter Verwendung einer starken Base umgesetzt. Die starke Base kann z. B. eine Alkalimetallhydroxid, wie Kalium- oder Natriumhydroxid, vorzugsweise in fester Form sein.

Eine bevorzugte Ausführungsform dieses Verfahrens besteht in der Umsetzung von Verbindungen der Formel IX mit Chloroform und Aceton in Gegenwart von gepulvertem Natriumhydroxid und der Siedetemperatur der Reaktionskomponenten.

Verbindungen der allgemeinen Formel I, in denen X eine Hydroxygruppe ist, lassen sich auch nach Verfahren f) herstellen, was dadurch gekennzeichnet ist, daß man eine Verbindung der allgemeinen Formel I, vorzugsweise I a, I b, oder I c, in der X = $OR^5$ ist verseift.

Die Verseifung führt man nach bekannten Standardverfahren in Gegenwart einer Base, wie Natrium-, Kalium-, Barium- oder Calciumhydroxid, Kaliumalkoholat durch. Als Lösungsmittel dienen niedere Alkohole, wie Methanol, Ethanol oder die verschiedenen Propanole oder deren Gemische mit Wasser.

Verbindungen der allgemeinen Formel I, in denen X für eine $R^5O$- oder $R^6R^7N$-Gruppe mit der für $R^5$,$R^6$ und $R^7$ oben genannten Bedeutung steht, lassen sich nach Verfahren g) herstellen, was dadurch gekennzeichnet ist, daß man eine Verbindung der Formel I, in der X eine Hydroxygruppe ist, mit dem entsprechenden Alkohol $R^5OH$, Amin oder Hydroxylamin $HNR^6R^7$ nach Verfahren kondensiert, die dem

Fachmann im Prinzip bekannt sind.

Dafür eigenen sich die in der Peptidchemie bewährten Kondensationsmittel wie zum Beispiel Carbonyldiimidazol, Dicyclohexylcarbodiimid, Diethoxyphosphonsäurechlorid, Diethoxyphosphonsäureazid, Phosphoroxychlorid, Propylphosphonsäureanhydrid und Diphenylphosphonsäurechlorid. Vorteilhafterweise führt man die Kondensation in einem Lösungmsittel durch. Geeignet sind, abhängig vom verwendeten Kondensationsmittel, nahezu alle gängigen organischen Lösungsmittel, wie Kohlenwasserstoffe (gesättig oder aromatisch), chlorierte Kohlenwasserstoffe, Ether, niedere Ketone wie Aceton oder Butanon, tert. Amide wie Dimethylformamid, Dimethylacetamid oder N-Methylpyrrolidon, niedere Alkohole wie Methanol, Ethanol, Isopropanol, n-, iso- oder tert.Butanol und sogar wäßrige Systeme bzw. Mischungen (homogen oder zweiphasig) der angeführten organischen Lösungsmittel mit Wasser.

Eine Ausführungsform dieses Verfahrens besteht in der Umsetzung der Verbindungen der allgemeinen Formel I, in denen X eine Hydroxygruppe ist, mit Dicyclohexylcarbodiimid in einem Lösungsmittel, besonders halogenierte Kohlenwasserstoffe wie Methylenchlorid oder Chloroform, gefolgt von der Zugabe eines basischen Katalysators wie 4-Diethylamino- oder Pyrrolidinopyridin und des Alkohols $R^5OH$ oder des Amins $HNR^6R^7$ bei Temperaturen von etwa 20 - 40 °C. Alternativ kann man die Carbonsäuren der Formel I mit X = OH zunächst in ein aktiviertes Derivat, wie Säurechlorid oder gemischtes Anhydrid überführen, und dieses dann mit einem Amin oder Hydroxylamin $HNR^6R^7$ umsetzten, vorzugsweise in Gegenwart einer Hilfsbase wie Natriumhydrogencarbonat, Natrium- bzw. Kaliumcarbonat, Natrium- bzw. Kaliumhydroxid oder einem tert. Amin wie Pyridin oder Triethylamin. Für die Aktivierung von Carbonsäuren sind dem Fachmann eine große Zahl von Methoden geläufig, z. B. die Umsetzung mmit Thionylchlorid, Phosphortrichlorid, Siliciumtetrachlorid, Phosgen oder Oxalylchlorid zum Säurechlorid oder die Reaktion mit Chlorameisensäureestern oder Sulfonsäurechloriden (Methansulfonsäurechlorid, Trifluormethansulfonsäurechlorid, Benzosulfonsäurechlorid) in Gegwenwart von Basen, vorzugsweise von tert. Aminen wie Triethylamin oder Pyridin, zu den gemischten Anhydriden.

Eine bevorzugte Variante dieser Ausführungsform des Verfahrens g) besteht in der Umsetzung einer Verbindung der Formel I, in denen $R^1$, $R^2$, A die oben angeführte Bedeutung hat und X eine Hydroxygruppe darstellt mit $HNR^6R^7$ in Gegenwart von Siliciumtetrachlorid in Pyridin bei Temperaturen von etwa -20 °C bis 40 °C, bevorzugt bei Raumtemperatur umsetzt.

Verbindungen der allgemeinen Formel I, in denen $R^1$, $R^2$ und A die oben angegebenen Bedeutung haben und X für eine $R^6R^7N$-Gruppe steht, lassen sich auch nach Verfahren h) herstellen, das dadurch gekennzeichnet ist, daß man eine Verbindung der allgemeinen Formel I, in der X ein $R^5O$-Rest darstellt, mit der entsprechenden Base $HNR^6R^7$ umsetzt.

Die Reaktion wird vorzugsweise in einem geeigneten organischen Lösungsmittel wie einem Alkohol (Methanol, Ethanol, n-Propanol, n-Butanol, Isopropanol, 2-Ethoxyethanol, 2-Methoxyethanol), einem Ether (bevorzugt Tetrahydrofuran, Dioxan, 1,2-Dimethyoxyethan, oder Diethylenglykoldimethylether) oder einem Kohlenwasserstoff, wie Xylol, Toluol, Mesitylen, Tetralin oder Dekalin durchgeführt. Man kann auch einen Überschuß des Amins oder Hydroxylamins als Lösungsmittel verwenden. Die Reaktion wird bei Temperaturen im Bereich von etwa 20 °C bis zum Siedepunkt des verwendeten Lösungsmittels durchgeführt, bevorzugt sind Temperaturen von etwa 40 °C bis 120 °C, besonders von etwa 40 bis 80 °C.

Verbindungen der allgemeinen Formel I a, in denen $R^1$, $R^2$ und $R^5$ die oben angegebene Bedeutung haben, $R^3$ ein Wasserstoffatom oder Methyl, $R^4$ ein Wasserstoffatom und Y eine Einfachbindung bedeutet, lassen sich auch nach Verfahren i) herstellen. Verfahren i) ist dadurch gekennzeichnet, daß man eine erfindungsgemäße Verbindung der allgemeinen Formel I b mit geeigneten Reduktionsmitteln umsetzt. Eine bevorzugte Ausführungsform ist die katalytische Hydrierung der Olefine über Palladium oder Platin auf Kohle als Katalysator in einem polaren Lösungsmittel wie Alkohole (Methanol, Ethanol) oder Essigsäure, bei Drücken von etwa 1 bis 3 atm und Temperaturen zwischen etwa 20 °C und 60 °C, bevorzugt bei Raumtemperatur.

Die erfindungegemäßen Verbindungen der allgemeinen Formel I können, soweit sie eine Carboxylgruppe enthalten, Salze mit anorganischen oder organischen Basen bilden. Daher sind auch solche Salze Gegenstand der vorliegenden Erfindung. Bevorzugt sind Salze mit anorganischen Basen, besonders die physiologisch unbedenklichen Alkalimetallsalze, vor allem Natrium- oder Kaliumsalze.

Die erfindungsgemäßen 2-substituierten 4-(3-Alkyl-5-tert.-butyl-4-hydroxyphenyl)-thiazole der Formel I und deren entsprechende Salze eignen sich aufgrund ihrer wertvollen pharmakologischen Eigenschaften in besonderer Weise für die Verwendung als Wirkstoffe in Arzneimitteln, insbesondere in solchen zur Behandlung von entzündlich-rheumatischen Erkrankungen. Sie könne entweder für sich allein, beispielsweise in Form von Mikrokapseln, in Mischungen miteinander oder in Kombination mit geeigneten Hilfs- und/oder Trägerstoffen verabreicht werden.

Gegenstand der Erfindung sind somit auch Arzneimittel, die aus mindestens einer Verbindung der

Formel I und/oder mindestens einem von deren entsprechenden Salzen bestehen oder mindestens einen dieser Wirkstoffe neben pharmazeutisch geeigneten und physiologisch verträglichen Trägerstoffen, Verdünnungsmitteln und/oder anderen Hilfsstoffen enthalten.

Die erfindungsgemäßen Arzneimittel könnnnen oral, systemisch, rektal oder gegebenenfalls auch parenteral appliziert werden, wobei die orale Anwendung bevorzugt ist.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyäthylenglykole und Lösungsmittel, wie etwa sterile Wasser und ein oder mehrwertige Alkohole, Z. B. Glycerin, genannt.

Vorzugsweise werden die pharmazeutischen Präparate in Dosierungseinnheiten hergestellt und verabreicht, wobei jede Einheit als aktiven Bestandteil eine bestimmmte Dosis mindestens einer Verbindung gemäß Formel I und/oder mindestens eines entsprechenden Salzes enthält. Bei festen Dosierungseinheiten, wie Tabletten, Kapseln, Dragess, oder Suppositorien, kann diese Dosis bis zu etwa 800 mg, bevorzugt jedoch etwa 100 bis 500 mg betragen.

Für die Behandlung eines an entzündlich- rheumatischen Erkrankungen leidenden, erwachsenen Patienten sind - je nach Wirksamkeit der Verbindungen gemäß Formel I und/oder der entsprechenden Salze am Menschen - Tagesdosen von etwa 100 bis 2000 mg Wirkstoff, vorzugsweise etwa 300 bis 1100 mg, bei oraler Verabreichung indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteiltet Dosen in bestimmten Intervallen erfolgen.

Schließlich können die Verbindungen der Formel I und die entsprechenden Salze bei der Herstellung der vorgenannten galenischen Zubereitungsformen auch zusammen mit anderen geeigneten Wirkstoffen, beispielsweise Antiuricopathika, Thrombocytenaggregationshemmern, Analgetika und anderen steroidalen oder nicht-steroidalen Antiphlogistika, formuliert werden.

Die Struktur aller nachstehend beschriebenen Verbindungen wurde durch Elementaranalyse und IR- sowie $^1$H-NMR-Spektren gesichert. Die gemäß nachfolgenden Beispielen und die auf analoge Weise hergestellten Verbindungen der Formel I sind in Tabelle 1 zusammengefaßt.

**Beispiel 1:**

3-[4-(3,5-Di-tert.-butyl-4-hydroxyphenyl)-thiazol-2-yl]-propionsäureethylester nach Verfahren a)

**$a_1$) 2-Brom-1-(3,5-di-tert.-butyl-4-hydroxyphenyl)-ethanon**

20,6 g (83 mmol) 1-(3,5-Di-tert.butyl-4-hydroxyphenyl)-ethanon wurden unter Rühren in 50 ml Methylenchlorid gelöst, zum Sieden erhitzt und innerhalb von 15 Minuten tropfenweise mit 14,4 g (90 mmol) Brom versetzt. Danach wurde für weitere 2 Stunden unter Rückfluß erhitzt, die Mischung abgekühlt, mit 50 ml Wasser versetzt, die organische Phase abgetrennt und über Natriumsulfat getrocknet. Nach dem Entfernen des Lösungsmittels unter vermindertem Druck wurde der feste Rückstand aus Methylcyclohexan umkristallisiert.

| | |
|---|---|
| Ausbeute: | 20,5 g (72 % der Theorie) |
| Schmelzpunkt: | 105 - 108 °C |
| | $C_{16}H_{23}BrO_2$ (MG = 327,3) |

**$a_2$) 3-(Ethoxycarbonyl)-thiopropionsäureamid**

In eine Lösung von 31,8 g (0,25mol) 3-Cyanopropionsäureethylester und 34,6 ml (0,25 mol) Triethylamin in 75 ml Pyridin wurde unter Rühren bei Raumtemperatur 9 Stunden Schwefelwasserstoff eingeleitet. Nach Stehenlassen über Nacht wird das Reaktionsgemisch unter Kühlung mit 2 N-Salzsäure sauer gestellt und mehrmals mit Essigsäureethylester extrahiert. Die vereinigte und über Natriumsulfat getrockneten organische Phase wird unter reduziertem Druck schonend eingeengt. Der verbleibende ölige Rückstand (35,0 g = 87 % d. Th.) besteht laut GC-Analyse aus ca. 87 % 3-(Ethoxycarbonyl)-thiopropionsäureamid und 13 % 3-Cyanpropionsäureethylester.

**$a_3$) 3-[4-(3,5-Di-Cert.-butyl-4-hydroxyphenyl)-thiazol-2-yl]-propionsäureethylester**

36,0 g (0,11 mol) 2-Brom-1-(3,5-di-tert.-butyl-4-hydroxyphenyl)-ethanon aus Stufe $a_1$) und 17,7 g (0.11 mol) 3-(Ethoxycarbonyl)-thiopropionsäureamid (reaktiver Bestandteil des aus Stufe $a_2$) gewonnenen Substanzgemisches) wurden in 100 ml Essigsäureethylester 1,5 Stunden unter Rückfluß erhitzt. Nach dem Abkühlen und Behandeln mit einer $NaHCO_3$-Lösung wurde die organische Phase abgetrennt, getrocknet und unter reduziertem Druck eingeengt. Ausrühren des verbleibenden öligen Rückstandes mit Petrolether (40 - 60° C) ergab einen kristallinen Niederschlag.

| | |
|---|---|
| Ausbeute: | 33,4 g (78 % der Theorie) |
| Schmelzpunkt: | 55 - 56° C |
| | $C_{22}H_{31}NO_3S$ (MG = 389,6) |

| | | | | | |
|---|---|---|---|---|---|
| Analyse: | Berechnet: | C 67,83 % | H 8,02 % | N 3,60 % | S 8,23 % |
| | Gefunden: | C 67,81 % | H 8,05 % | N 3,57 % | S 8,44 % |

Die Verbindung wurde auch nach Verfahren i) durch katalytische Hydrierung von 3-[4-(3.5-Di-tert.butyl-4-hydroxyphenyl)-thiazol-2-yl]-acroleinsäureethylester wie folgt hergestellt:
In einer Parr-Apparatur wurde eine Lösung von 6 g (15,5 mmol) 3-[4-(3,5-Di-tert.butyl-4-hydroxyphenyl)-thiazol-2-yl]-acroleinsäureethylesters (dessen Herstellung siehe Beispiel 2) in 200 ml Eisessig in Gegenwart von 2 g Palladium/Kohle (10 %) hydriert, bis kein Wasserstoff mehr aufgenommen wurde. Abfiltration des Katalysators und Einengen der Mischung unter reduziertem Druck ergab einen Rückstand, der wie im Falle von $a_3$) zuerst in Essigester aufgenommen, mit $NaHCO_3$-Lösung behandelt und schließlich nach Eindampfen des Lösungsmittels zu einem öligen Rückstand führte. Aus diesem fiel nach Behandlung mit Petrolether das Produkt als farblose Kristalle an.

| | |
|---|---|
| Ausbeute: | 4,3 g (71 % der Theorie) |
| Schmelzpunkt: | 56 - 57° C |
| | $C_{22}H_{31}NO_3S$ (MG = 389,6) |

Analytische und spektroskopische Daten bestätigen die Identität des gewonnen Produktes mit der nach Verfahrensweise a) hergestellten Verbindung.

**Beispiel 2**
3-[4-(3,5-Di-tert.butyl-4-hydroxyphenyl)-thiazol-2-yl-]-acroleinsäureethylester
nach Verfahren b)
**$b_1$) 4-(3,5-Di-tert.-butyl-4-hydroxyphenyl)-2-formyl-thiazol**

90 g (0,27 mol) 2-Brom-1-(3,5-di-tert.butyl-4-hydroxyphenyl)-ethanon aus Stufe a1) und 42.4 g (0,26 mol) Diethoxythioacetamid wurden in 200 ml Ethanol 30 Minuten bei Raumtemperatur gerührt. Nach Entfernen des Lösungmittels unter reduziertem Druck wurde der Rückstand mit 200 ml Essigsäureethylester in der Wärme behandelt. Der dabei kristallin anfallende Niederschlag an entsprechendem Diethylacetat, wurde zur Freisetzung der Aldehydgruppe in 1500 ml Aceton gelöst, mit 230 ml 4 N Salzsäure versetzt und 2 Stunden bei Raumtemperatur gerührt. Nach Neutralisation mit $Na_2CO_3$-Lösung fällt ein Niederschlag aus, der nach dem Abfiltrieren nochmals aus Petrolether umkristallisiert wurde.

| | |
|---|---|
| Ausbeute: | 63,4 g (77 % der Theorie) |
| Schmelzpunkt: | 99 - 100° C |
| | $C_{18}H_{23}NO_2S$ (MG = 317,5) |

| | | | | | |
|---|---|---|---|---|---|
| Analyse: | Berechnet: | C 68,10 % | H 7,30 % | N 4,41 % | S 10,10 % |
| | Gefunden: | C 67,86 % | H 7,29 % | N 4,35 % | S 9,97 % |

$b_2$) 3-[4-(3,5-Di-tert.butyl-4-hydroxyphenyl)-thiazol-2-yl]-acroleinsäureethylester

Zu einer Lösung von 31,7 g (0,1 mol) des Aldehyds aus Stufe $b_1$) und 23,3 g (0,1 mol) Phosphonoessigsäuretriethylester in 400 ml trockenem Dimethylformamid trägt man unter Rühren portionsweise 6.6 g

(0,22 mol) 80 %-iges Natriumhydrid ein, wobei die Reaktionstemperatur durch Wasserkühlung unter 30 °C gehalten wird. Nach 3-stündigem Rühren bei Raumtemperatur wird nach Zugabe von 375 ml 4-N Schwefelsäure mehrmals mit Essigsäureethylester extrahiert. Die vereinigten Ester-Phasen werden 2 mal mit jeweils 300 ml gesättigter $NaHCO_3$-Lösung ausgeschüttelt, mit Wasser gewaschen, über Natriumsulfat getrocknet und im Vakuum eingeengt. Umkristallisation des Rückstandes aus Methanol ergab gelbe Kristalle.

| | |
|---|---|
| Ausbeute: | 33,3 g (86 % der Theorie) |
| Schmelzpunkt: | 126 - 127 °C |
| | $C_{22}H_{29}NO_3S$ (MG = 387,5) |

| | | | | | |
|---|---|---|---|---|---|
| Analyse: | Berechnet: | C 68,18 % | H 7,54 % | N 3,61 % | S 8,27 % |
| | Gefunden: | C 67,87 % | H 7,66 % | N 3,59 % | S 8,31 % |

**Beispiel 3:**
3-[4-(3,5-Di-tert.-butyl-4-hydroxyphenyl)-thiazol-2-yl)-propionsäure
**nach Verfahrensweise f)**

Zu einer Lösung von 7,5 g (19 mmol) des Esters aus Beispiel 1 in 35 ml Ethanol wurde nach Zugabe von 6 ml 10 N-Natronlauge 30 Minuten bei Raumtemperatur gerührt. Danach wurde mit 4 N-Salzsäure auf pH 1 gestellt. Die dabei anfallenden Kristalle wurden abgesaugt, und zwischen Essigsäureethylester und Wasser verteilt. Die abgetrennte organische Phase wurde nach dem Trocknen im Vakuum eingeengt und der Rückstand aus Petrolether/Diisopropylether (10 : 1) umkristallisiert.

| | |
|---|---|
| Ausbeute: | 5,9 g (86 % der Theorie) |
| Schmelzpunkt: | 154 - 155 °C |
| | $C_{20}H_{27}NO_3S$ (MG = 361,5) |

| | | | | | |
|---|---|---|---|---|---|
| Analyse: | Berechnet: | C 66,45 % | H 7,53 % | N 3,87 % | S 8,87 % |
| | Gefunden: | C 66,55 % | H 7,75 % | N 3,86 % | S 8,68 % |

**Beispiel 4:**
3-[4-(3,5-Di-tert.-butyl-4-hydroxyphenyl)-thiazol-2-yl-methyliden]-pyrrolidin-2-on
**nach Verfahrensweise c)**

16,0 g (91 mmol) 3-Brompyrrolidin-2-on wurden in 30 ml Essigsäureanhydrid 1 Stunde zum Sieden erhitzt. Nach dem Eindampfen des Reaktionsgemisches unter reduziertem Druck zur Trockne wurde der Rückstand in 50 ml Tetrahydrofuran gelöst und nach Zugabe von 26,2 g (0,1 mol) Triphenylphosphin 5 Stunden unter Rückfluß erhitzt. Danach wurde erneut eingeengt, der Rückstand in 300 ml Ethanol gelöst und mit 24,7 g (78 mmol) des Aldehyds aus Beispiel 2 $b_1$) und 27 ml (0,36 mol) Triethylamin versetzt. Nach 2-stündigem Erwärmen des Gemisches auf 70 °C wurde der gelbe Niederschlag abfiltriert und mit Ethanol gewaschen. Man löst den Kristallbrei in etwa 2 l Chloroform auf, wäscht mehrmals mit gesättigter Kochsalzlösung, trocknet über $Na_2SO_4$ und engt die Chloroformphase auf ein Drittel ein. Beim längeren Stehenlassen scheiden sich gelbe Kristalle aus.

| | |
|---|---|
| Ausbeute: | 24,7 g (81 % der Theorie) |
| Schmelzpunkt: | 244 - 245 °C |
| | $C_{22}H_{28}N_2O_2S$ (MG = 384,5) |

| | | | | | |
|---|---|---|---|---|---|
| Analyse: | Berechnet: | C 68,72 % | H 7,34 % | N 7,28 % | S 8,34 % |
| | Gefunden: | C 68,45 % | H 7,13 % | N 6,89 % | S 7,97 % |

**Beispiel 5:**
2-[4-(3,5-Di-tert.-butyl-4-hydroxyphenyl)-thiazol-2-yl-methyliden)-aminooxy]-essigsäure
**nach Verfahrensweise d)**

Zu einer Lösung von 15,9 g (0,05 mol) des Aldehyds aus Beispiel 2 $b_1$) in 200 ml Methanol tropft man gleichzeitig eine Lösung von 6,4 g (0,05 mol) Carboxymethoxyaminhydrochlorid in 20 ml Wasser und 2 g (0.05 mol) Natriumhydroxid in 200 ml Wasser unter Rühren zu. Nach weiterem 1,5-stündigen Rühren bei 50 °C, wird das Methanol weitgehend im Vakuum abdestilliert und der Rückstand mehrfach mit Essigsäureethylester extrahiert. Die vereinigten Extrakte werden mit Wasser gewaschen, über $Na_2SO_4$ getrocknet und im Vakuum eingedampft. Der ölige Rückstand wird nach Behandeln mit Petrolether (40 - 60 °C) kristallin.

Ausbeute: 13,8 g (71 % der Theorie)
Schmelzpunkt: 178 - 180 °C
$C_{20}H_{26}N_2O_4S$ (MG = 390,5)

| Analyse: | | | | | |
|---|---|---|---|---|---|
| | Berechnet: | C 61,52 % | H 6,71 % | N 7,17 % | S 8,21 % |
| | Gefunden: | C 61,44% | H 6,85 % | N 6,96 % | S 8,02 % |

**Beispiel 6:**
2-[4-(3,5-Di-tert.-butyl-4-hydroxyphenyl)-thiazol-2-yl)-methoxy]-2-methylpropionsäure
**nach Verfahrensweise d)**
$d_1$) [4-(3,5-Di-tert.-butyl-4-hydroxyphenyl)-2-hydroxymethyl-thiazol

117,8 g (0,36 mol) 2-Brom-1-(3,5-di-tert.-butyl-4-hydroxyphenyl)-ethanon und 72,1 g (0,34 mol) Benzoyloxythioacetamid werden in 300 ml Ethanol gelöst und 4 Stunden bei Raumtemperatur gerührt. Die dabei sich bildenden Kristalle werden abfiltriert und mit wenig Ethanol gewaschen. Zur Freisetzung der Hydroxygruppe wurden 139,4 g des isolierten Benzoylesters (Fp.: 198 - 199 °C als Hydrobromid) in 600 ml Ethanol aufgenommen, mit 45,6 g (0,69 mol) 85 %-igem Kaliumhydroxid in 30 ml Wasser versetzt und eine halbe Stunde bei Raumtemperatur nachgerührt. Danach wird die Mischung eingeengt, mit Wasser versetzt und mehrfach mit Essigester extrahiert. Der vereinigten Extrakte werden nach dem Trocknen über $Na_2SO_4$ mit ethanolischer Salzsäure versetzt, wobei der Alkohol in Form des Hydrochlorides ausfällt.

Ausbeute: 78,6 g (65 % der Theorie)
Schmelzpunkt: 185 - 186 °C (als Hydrochlorid)
$C_{18}H_{26}ClNO_2S$ (MG = 355,9)

Analyse:

| | | | | | |
|---|---|---|---|---|---|
| Berechnet: | C 60,74 % | H 7,36 % | Cl 9,96 % | N 3,94 % | S 9,01 % |
| Gefunden: | C 60,49 % | H 7,56 % | Cl 10,05 % | N 6,96 % | S 9,05 % |

$d_2$) 2-[4-(3,5-Di-tert.-butyl-4-hydroxyphenyl)-thiazol-2-yl)-methoxy]-2-methylpropionsäure

9,6 g (0,031 mol) des Alkohols aus Stufe $e_1$) in Form der freien Base werden mit 6.0 g (0,15 mol) gepulvertem Natriumhydroxid in 44 ml Aceton vermischt und die Suspension zum Rückfluß erhitzt. Dann werden 4,8 g (0,04 mol) Chloroform in 10 ml Aceton zugetropft und weitere 5 Stunden bei Rückflußtemperatur erhitzt. Danach werden die Lösungsmittel im Vakuum abgedampft und der Rückstand zwischen Diisopropylether und Wasser verteilt. Die organische Phase wird abgetrennt und die wässrige Phase nach nochmaligem Extrahieren mit Ether mit konzentrierter Salzsäure auf pH 2 angesäuert und mehrmals mit Methylenchlorid extrahiert. Die vereinigten Methylenchloridextrakte werden getrocknet, filtriert und eingeengt. Der Rückstand wird durch Säulenchromatographie an Kieselgel (90 - 130 ) Laufmittel: Methylenchlorid/Methanol (50 : 1) gereinigt, mit wenig Petrolether (40 - 60 °C) zur Kristallisation gebracht.

Ausbeute: 3,1 g (26 % der Theorie)
Schmelzpunkt: 158 - 159 °C
$C_{22}H_{31}NO_4S$ (MG = 405,6)

| | | | | | |
|---|---|---|---|---|---|
| Analyse: | Berechnet: | C 65,16 % | H 7,70 % | N 3,45 % | S 7,91 % |
| | Gefunden: | C 65,03 % | H 7,89 % | N 3,37 % | S 7,81 % |

**Beispiel 7:**
3-[4-(3,5-Di-tert.-butyl-4-hydroxyphenyl)-thiazol-2-yl]-propionhydroxamsäure
**nach Verfahrensweise g)**

Zu einer Lösung von 6,7 g (18,6 mmol) der Säure aus Beispiel 3) in 250 ml Methylenchlorid gibt man bei 0 °C zuerst 1,4 g Dimethylformamid, dann 5,3 g (42 mmol) Oxalsäuredichlorid und rührt 1 Stunde nach. Beim Zutropfen einer Lösung von 7,7 g (111 mmol) Hydroxylamin-hydrochlorid in 65 ml THF und 13 ml Wasser und 11,2 g (111 mmol) Triethylamin läßt man die Reaktionstemperatur auf 30 °C steigen und rührt weitere 12 Stunden nach. Man fügt anschließend 300 ml 2-N Salzsäure zu und trennt die organische Phase ab. Die wässrige Phase wird nochmals mit Methylenchlorid extrahiert und die vereinigten organischen Phasen mit ges. $NaHCO_3$- und NaCl-Lösung gewaschen, über $Na_2SO_4$ getrocknet und im Vakuum eingeengt. Nach Zugabe von Petrolether (40 - 60 °C) verfestigt sich der Rückstand.

| | |
|---|---|
| Ausbeute: | 3,8 g (55 % der Theorie) |
| Schmelzpunkt: | 182 - 183 °C |
| | $C_{20}H_{28}N_2O_3S$ (MG = 376,6) |

| | | | | | |
|---|---|---|---|---|---|
| Analyse: | Berechnet: | C 63,80 % | H 7,50 % | N 7,44 % | S 8,52 % |
| | Gefunden: | C 63,95 % | H 7,61 % | N 7.,41 % | S 8,63 % |

**Tabelle 1:** Verbindungen gemäß Formel I (siehe Anspruch 1)

| Beispiel | $R^1$ | $R^2$ | A | X | Schmelz-punkt °C |
|---|---|---|---|---|---|
| 1 | $(H_3C)_3C-$ | H | $-(CH_2)_2-$ | $-OC_2H_5$ | 55 - 57 |
| 2 | $(H_3C)_3C-$ | H | -CH=CH- | $-OC_2H_5$ | 126 - 127 |
| 3 | $(H_3C)_3C-$ | H | $-(CH_2)_2-$ | -OH | 154 - 155 |
| 4 | (H3C)3C- | H | -CH= 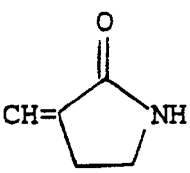 | | 244 - 245 |
| 5 | $(H_3C)_3C-$ | H | $-CH=N-O-CH_2-$ | -OH | 178 - 180 |
| 6 | $(H_3C)_3C-$ | H | 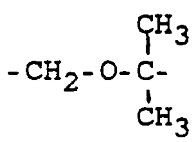 | -OH | 158 - 159 |
| 7 | $(H_3C)_3C-$ | H | $-(CH_2)_2-$ | -NH-OH | 182 - 183 |
| 8 | $(H_3C)_3C-$ | H | $-CH_2-$ | $-O-C_2H_5$ | 79 - 80 |
| 9 | $(H_3C)_3C-$ | H | $-CH_2-$ | -OH | 88-89 (Zers.) |
| 10 | $(H_3C)_3C-$ | H | $-(CH_2)_3-$ | -OH | 123 - 124 |
| 11 | $(H_3C)_3C-$ | H | -CH=CH- | -OH | 195 - 196 |
| 12 | $(H_3C)_3C-$ | H | -CH=CH- | $-OCH_3$ | 129 - 130 |
| 13 | $(H_3C)_3C-$ | H | $-(CH_2)_2-$ | $-OCH_3$ | 77 - 78 |

| Beispiel | R1 | R2 | A | X | Schmelzpunkt °C |
|---|---|---|---|---|---|
| 14 | $(H_3C)_3C-$ | $CH_3$ | $-(CH_2)_2-$ | $-OC_2H_5$ | 67 - 68 |
| 15 | $(H_3C)_3C-$ | $CH_3$ | $-(CH_2)_2-$ | $-OH$ | 132 - 133 |
| 16 | $CH_3-$ | H | $-(CH_2)_2-$ | $-OC_2H_5$ | 127 - 129 |
| 17 | $CH_3-$ | H | $-(CH_2)_2-$ | $-OH$ | 138 - 140 |
| 18 | $(H_3C)_3C-$ | H | $-CH_2-S-CH_2-$ | $-OH$ | 146 - 148 |
| 19 | $(H_3C)_3C-$ | H | $-(CH_2)_2-$ | $-N(CH_3)OH$ | 79 - 80 |
| 20 | $(H_3C)_3C-$ | H | $-(CH_2)_2-$ | $-NHOCH_3$ | 160 - 161 |
| 21 | $(H_3C)_3C-$ | H | $-(CH_2)_2-\overset{H}{N}-$ | 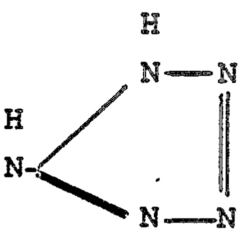 | 232 - 233 |
| 22 | $(H_3C)_3C-$ | H | $-(CH_2)_2-$ | $-N(C_2H_5)_2$ | 116 - 117 |
| 23 | $(H_3C)_3C-$ | H | $-(CH_2)_2-$ | $-O-(CH_2)_2-OC_2H_5$ | Öl |

**Pharmakologische Prüfung und Ergebnisse**

Die Prüfung der erfindungsgemäßen Verbindungen der Formel I auf antiphlogistische Wirkung, Beeinflussung immunpathologischer Prozesse, Sauerstoffradikale desaktivierende Eigenschaften und Beeinflussung des Arachidonsäuremetabolismus erfolgte in den anschließend beschriebenen Tiermodellen und Bioassays.

**1. Adjuvans-Arthritis zur Beeinflussung der antiphlogistischen Wirkung (Tabelle 2)**

Die Untersuchungen wurden nach der Methode von Pearson (Arthrit. Rheum. 1959, 2, 44) durchgeführt. Als Versuchstiere dienten männliche Ratten eines Wistar-Lewis-Stammes mit einem Körpergewicht zwischen 130 und 200 g. Die zu prüfenden Verbindungen wurden in Dosen von 50 mg pro kg Körpergewicht einmal täglich vom 1. bis 5. Versuchstag oral (p. o.) appliziert. Die Tiere einer Kontrollgruppe erhielten nur

das Vehikel. Jede Präparat- und und die Kontrollgruppe umfaßte 8 Tiere. Als Wirkungskriterium diente die prozentuale Herabsetzung der Pfotenvolumenzunahme gegenüber jener der unbehandelten Kontrollgruppe. Die $ED_{50}$-Werte wurden graphisch aus der Dosiswirkungskurve bestimmt.

Als Vergleichspräparat wurde 4-(3,5-Di-tert.butyl-4-hydroxyphenyl)-2-methylthiazol - d.i. die Verbindung der Formel

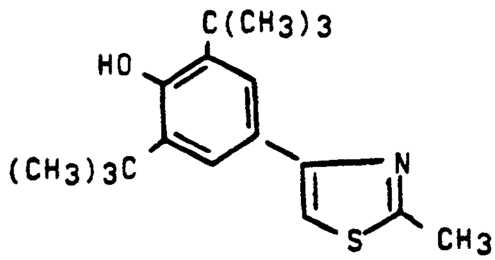

gemäß der anfangs erwähnten US-A-4 535 165, in diese und die nachstehende Untersuchung mit einbezogen.

**Tabelle 2: Antiphlogistische Wirkung am 5. Tag**

| Verbindung aus Beispiel | Adjuvans-Arthritis (% Hemmung bei 50 mg/kg per os) | $ED_{50}$-Wert |
|---|---|---|
| 1 | 73 | 5,7 |
| 3 | 84 | 0,9 |
| 4 | 54 | |
| 5 | 51 | |
| 6 | 70 | |
| 7 | 83 | 2,4 |
| 10 | 71 | |
| 11 | 63 | 12,6 |
| 13 | 74 | |
| 14 | 61 | |
| 15 | 67 | 2,6 |
| 17 | 67 | 7,5 |
| 19 | 75 | 2,5 |
| 20 | 66 | |
| 21 | 65 | |
| Vergleichs-präparat | 67 | |

**2. Hemmung immunpathologischer Prozesse**

Es ist allgemein anerkannt, daß der progrediente Verlauf der entzündlich rheumatischen Erkrankungen hauptsächlich durch Entgleisungen des Immunsystems verursacht wird und somit eine mehr kausale

Therapie nur mit solchen Medikamenten gelingen kann, die diese immunpathologischen Prozesse hemmen.

a) Adjuvans Arthritis (Perper-Modifikation)

In der unter Punkt 1 beschriebenen Versuchsanordnung wurden die Tiere lediglich vom 1. bis 12. Versuchstag behandelt. Nach einem behandlungsfreien Intervall von 9 Tagen erfolgt die Bestimmung des Pfotenvolumens der linken und rechten Hinterpfote am 21. Tag (vergl. Tabelle 3). In diesem Test sind klassische nicht-steroidale Antiphlogsitika unwirksam, weil sie außerstande sind, die der chronischen Entzündungsphase zugrunde liegenden, immunpathologischen Prozesse zu hemmen. Desweiteren belegen die Ergebnisse eindeutig die Überlegenheit der erfindungsgemäßen Verbindungen der Formel I gegenüber dem Vergleichspräparat aus US-A-4 535 165, welches in der Perper-Modifikation des Adjuvans Arthritis nur eine angedeutete Wirkung zeigt.

**Tabelle 3**

| Verbindung aus Beispiel | Dosierung mg/kg/Tag | % Hemmung am 21. Tag linke Pfote | rechte Pfote |
|---|---|---|---|
| 1 | 50 | 45 | 49 |
| 3 | 2,5 | 65 | 59 |
| | 5 | 41 | 74 |
| 5 | 50 | 20 | 42 |
| 11 | 50 | 44 | 51 |
| 13 | 50 | 44 | 31 |
| 14 | 50 | 22 | 49 |
| 15 | 50 | 39 | 50 |
| 17 | 50 | 60 | 38 |
| 19 | 5 | 59 | 70 |
| | 10 | 72 | 85 |
| 20 | 50 | 50 | 35 |
| 21 | 50 | 42 | 54 |
| Vergleichs-präparat | 50 | 12 | 21 |

b) Reverse passive Arthus-Reaktion

Als Versuchstiere dienten männliche Sprague-Dawley-Ratten mit einem Körpergewicht zwischen 100 und 120 g, die in Gruppen zu jeweils 8 Tieren aufgeteilt wurden. Die Tiere erhielten 1 Stunde nach oraler Verabreichung der Prüfsubstanz 0,5 mg Immunglobulin in 0,1 ml einer Natriumchlorid-Lösung subplantar in die linke Hinterpfote injiziert. Nach 4 Stunden wurde die Arthus-Reaktion gemessen, wobei als Meßparameter für die Wirkung die prozentuale Veränderung der Pfotenvolumenzunahme gegenüber jener der nur mit Vehikel behandelten Kontrollgruppe diente.

Nach Tabelle 4 zeichnen sich beispielsweise folgende erfindungsgemäßen Verbindungen als starke Hemmer der Arthus-Reaktion aus:

**Tabelle 4**

| Verbindung aus Beispiel | Dosierung mg/kg per. os. | % Hemmung |
|---|---|---|
| 5 | 60 | 38 |
| 8 | 60 | 38 |
| 18 | 100 | 50 |
| 19 | 50 | 45 |
| 15 | 100 | 47 |
| 21 | 50 | 48 |

**3. Wirkung als Radikalfänger und als Inhibitoren des Arachidonsäuremetabolismus**

a) Radikalfängereigenschaften

Die Prüfung in diesem Test, der auf ein antioxidatives Potential einer Substanz schließen läßt, erfolgte gemäß Smith et al., Biochem. Pharmacol. 1987, 36, 1456. Dabei wird die Reaktion der erfindungsgemäßen Verbindungen mit dem stabilen Radikal 1,1-Diphenyl-2-picryl-hydrazyl (DPPH) bei 20 °C optisch verfolgt. Die Geschwindigkeitskonstante K und Reaktionsordnungen n in Tabelle 5 wurden in üblicher Weise graphisch bestimmt.

**Tabelle 5**

| Verbindungen aus Beispiel | k | n |
|---|---|---|
| 1 | 2,24 | <1 |
| 2 | 0,39 | 1 |
| 3 | 0,70 | <1 |
| 7 | 0,44 | 1 |
| 8 | 0,30 | 1 |
| 9 | 0,46 | <1 |
| 11 | 0,16 | 1 |
| 12 | 0,51 | 1 |
| 13 | 0,07 | 1 |
| 15 | 3,50 | <1 |
| 16 | 1,22 | <1 |
| 18 | 0,42 | 1 |
| 19 | 6,60 | <1 |
| 21 | 0,43 | 1 |

b) Arachidonsäuremetabolismus

Die Hemmwirkung der erfindungsgemäßen Verbindungen wird auf den durch Cyclooxygenase und

Lipoxygenase katalysierten Arachidonsäure-Abbau in vitro mit Hilfe der in Weithmann und Alpermann, Arzneim.-Forsch. 1985, 35, 947 beschriebenen Testsysteme gemessen:

Im mikrosomalen Cyclooxygenase-System (Fraktion aus Schafsamenblasen, Fa. Paesel, Frankfurt, Deutschland) wird die durch Cyclooxygenase katalysierte Synthese von Prostaglandinen aus Arachidonsäure gemessen. Coenzym ist Adrenalin, dessen Umwandlung zu Adrenochrom bei 492 nm spektralphotometrisch verfolgt wird. Im Lipoxygenase-System in vitro wird Cis-9-cis-12-Linolsäure mit Lipoxygenase (L7127, Fa. Sigma, Deisenhofen, Deutschland) inkubiert und die bei der Oxygenierungsreaktion erfolgte Bildung von konjugierten Doppelbindungen optisch bei 234 nm verfolgt.

Die Inhibitorwirkungen, bzw. die für eine 50 %ige Hemmung der Enzymaktivität erforderlichen Inhibitorkonzentrationen ($IC_{50}$-Werte) sind für folgende erfindungsgemäße Beispiele ermittelt worden:

**Lipoxygenase (Enzym)**

Beispiel 8: $IC_{50}$ = 50 µM

Beispiel 12: bei 50 µM: 73 % Hemmung

Beispiel 15: bei 100 µM: 78 % Hemmung

**Cyclooxygenase (Mikrosomen)**

Beispiel 7: bei 100 µM: 65 % Hemmung

Beispiel 8: bei 100 µM: 75 % Hemmung

Beispiel 16: $IC_{50}$ = 34 µM

Darüber hinaus wurden die erfindungsgemäßen Substanzen als Inhibitoren der Arachidonsäure-Metabolite auch in zellulären Leukozyten-Systemen in vitro charakterisiert. Zum Nachweis der Lipoxygenase-Metabolite wurden durch Calcium-Ionophor A 23 187 (70 mcmol/l) stimulierte Human-Neutrophilen mit $^{14}C$-Arachidonsäure (81 mcmol/l) inkubiert und die nach 15 Minuten bei 37° C gebildeten Hauptmetabolite, wie 5-Hydroxyeicosatetraensäure (5-HETE) und das noch stärker proinflammatorisch wirkende Leukotrien $B_4$ - ($LTB_4$) nach Auftrennung durch HPLC mit Hilfe eines Radio-Monitors quantitativ bestimmt (Tab. 6). In entsprechender Weise wurde der Einfluß auf die proinflammatorischen Arachidonsäure-Metaboliten Thromboxan und $LTB_4$ in aus Ratten-Mastzellen hergestellten Kulturen bestimmt (Tab. 7).

**Tabelle 6**

Einfluß der Verbindungen gemäß Beispielen 7 und 9 auf die Aktivität der $LTB_4$- bzw. 5-HETE-Bildung in Human-Neutrophilen. (n = Anzahl der Messungen).

| | | $LTB_4$-Bildung | | |
|---|---|---|---|---|
| Beispiel 7 Inhibitor Konzentration | 100 µM | 10 µM | 1 µM | 0.1 µM |
| n | 9 | 6 | 6 | 6 |
| Restaktivität(%) | 1,7±1,3 | 35±17 | 74,3±21,5 | 91,0±13,2 |

| | | 5-HETE-Bildung | | |
|---|---|---|---|---|
| Beispiel 7 Inhibitor Konzentration | 100 µM | 10 µM | 1 µM | 0.1 µM |
| n | 9 | 6 | 6 | 6 |
| Restaktivität(%) | 1,7±0,4 | 39±12,8 | 87,0±15,6 | 89,2±12,1 |

| | | $LTB_4$-Bildung | | |
|---|---|---|---|---|
| Beispiel 9 Inhibitor Konzentration | 100 µM | 10 µM | 1 µM | 0.1 µM |
| n | 9 | 6 | 6 | 6 |
| Restaktivität(%) | 20,5±11 | 73±12 | 79±11 | 70±12 |

| | | 5-HETE-Bildung | | |
|---|---|---|---|---|
| Beispiel 9 Inhibitor Konzentration | 100 µM | 10 µM | 1 µM | 0.1 µM |
| n | 9 | 6 | 6 | 6 |
| Restaktivität(%) | 21±8 | 75±13 | 80±8 | 91,5±10 |

**Tabelle 7**

**Einfluß verschiedener Konzentrationen der Verbindung gemäß Beispiel 3 auf die Bildung von $TXB_2$ und $LTB_4$ in Mastzell-Kulturen (Ratte).**

| Beispiel 3 | $TXB_2$ | $LTB_4$ |
|---|---|---|
| | Restaktivität (%) | |
| 100 µM | 28±10 | 78±14 |
| 10 µM | 94±17 | 102± 9 |
| 1 µM | 113±49 | 94±12 |

**Ansprüche**

1. 2-substituierte 4-(3-Alkyl-5-tert.-butyl-4-hydroxyphenyl)-thiazole der allgemeinen Formel I, in der

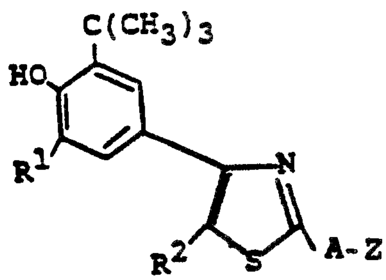

(I)

$R^1$
eine gesättigte oder ungesättigte, geradkettige oder verzweigte $C_1$-$C_5$-Alkylgruppe,
$R^2$
ein Wasserstoffatom oder ein Alkylrest mit 1 bis 3 C-Atomen,
A
eine Zwischenkette der Formel

$-(CH_2)_n-Y-CR^3R^4-$; $-CH=CR^3-(CH_2)_m-$ oder $-CH=N-O-(CH_2)_n-$

wobei Y eine Einfachbindung, ein Sauerstoff- oder Schwefelatom oder eine Carbonylgruppe, $R^3$ und $R^4$ gleich oder verschieden sind und ein Wasserstoffatom oder einen Alkylrest mit bis zu 2 C-Atomen, m eine Zahl von 0 bis 3 und n eine Zahl von 1 bis 4 darstellen, und Z
eine Tetrazol-, CN-Gruppe oder einen Rest der Formel

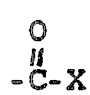

X
eine Hydroxygruppe oder ein Rest der allgemeinen Formel $R^5O$- oder $R^6R^7N$- , wobei $R^5$ ein geradkettiger oder verzweigter, gegebenfalls durch Hydroxy, $C_1$-$C_3$-Alkoxy oder $C_1$-$C_3$-Alkylamino-

substituierter $C_1$-$C_4$-Alkylrest, $R^6$ und $R^7$ gleich oder verschieden sind und ein Wasserstoffatom, ein geradkettig oder verzweigter $C_1$-$C_6$-Alkylrest, oder für den Fall, daß $R^6$ ein Wasserstoffatom oder ein $C_1$-$C_4$-Alkylrest bedeutet, $R^7$ eine Hydroxy-, $C_1$-$C_3$-Alkoxy-oder eine Tetrazol-5-yl-gruppe oder X mit den Strukturelementen -A-(C = O)- zusammen einen Rest der allgemeinen Formel II darstellt,

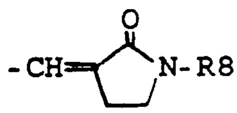

(II)

wobei $R^8$ ein Wasserstoffatom, einen $C_1$-$C_3$-Alkyl- oder $C_1$-$C_3$-Alkoxyrest bedeutet,
sowie physiologisch verträgliche Salze solcher Verbindungen der allgemeinen Formel I, in denen X eine Hydroxy- oder Hydroxyaminogruppe darstellt.

**2.** Verbindungen gemäß Anspruch 1 der allgemeinen Formel I, in denen
$R^1$
eine tert.-Butyl- oder Methylgruppe darstellt,
$R^2$
für Wasserstoff oder Methyl,
A
für eine Zwischenkette der Formel

$-(CH_2)_n$-Y-$CR^3R^4$-

steht, wobei Y eine Einfachbindung oder ein Sauerstoffatom, $R^3$ und $R^4$ gleich oder verschieden sind und ein Wasserstoffatom oder einen Methylrest, und n eine Zahl von 1 oder 2 darstellen, und
X
eine Hydroxygruppe oder ein Rest der allgemeinen Formel $R^5O$- oder $R^6R^7N$- darstellt, wobei $R^5$ ein geradkettiger oder verzweigter $C_1$-$C_3$-Alkylrest, $R^6$ ein Wasserstoffatom oder ein Methylrest und $R^7$ eine Hydroxy-, Methoxy- oder Tetrazol-5-yl-gruppe bedeutet.

**3.** Verbindungen gemäß Anspruch 2 der allgemeinen Formel I, in denen
$R^1$
für eine tert.-Butylgruppe
$R^2$
für Wasserstoff oder Methyl,
A
für eine Zwischenkette der Formel

$-(CH_2)_n$-Y-$CR^3R^4$-

steht, wobei Y eine Einfachbindung, $R^3$ und $R^4$ ein Wasserstoffatom und n eine Zahl von 1 oder 2 darstellen und
X
eine Hydroxygruppe oder $R^6R^7N$-Rest darstellt, wobei $R^6$ ein Wasserstoffatom oder ein Methylrest und $R^7$ eine Hydroxy-, oder Methoxygruppe bedeutet.

**4.** Verbindung nach Anspruch 3, dadurch gekennzeichnet, daß sie 3-[4-(3,5-Di-tert.-butyl-4-hydroxyphenyl)-thiazol-2-yl]-propionsäure darstellt.

**5.** Verfahren zur Herstellung von 2-substituierten 4-(3-tert.-Butyl-4-hydroxyphenyl)-thiazole der Formel I gemäß einem oder mehreren der Ansprüche 1 bis 4 und gegebenenfalls ihrer physiologisch verträglichen Salze, dadurch gekennzeichnet, daß man
a) ein Thiacarbonsäureamid der allgemeinen Formel III

$$H_2N-\overset{S}{\overset{\|}{C}}-(CH_2)_n-Y-CR^3R^4-\overset{O}{\overset{\|}{C}}-OR^5 \qquad (III)$$

mit 2-Halogen-1-phenylalkanonen der Formel IV,

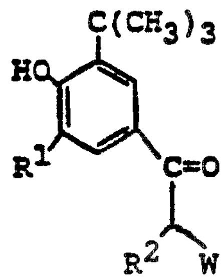

(IV)

in der $R^1$ und $R^2$ die in Anspruch 1 genannte Bedeutung haben und W für ein Halogen, vorzugsweise Chlor oder Brom steht, zu den erfindungsgemäßen Verbindungen der Formel Ia,

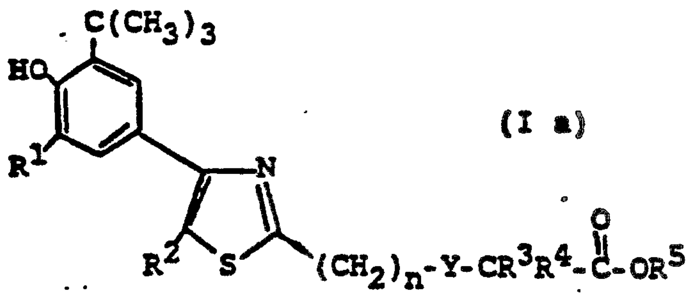

(I a)

mit den für $R^1$, $R^2$, $R^3$, $R^4$, $R^5$, Y und n in Anspruch 1 angegebenen Bedeutungen, umsetzt, oder b) einen Thiazolaldehyd der allgemeinen Formel V,

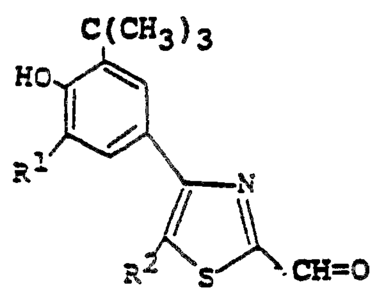

(V)

in einer Olefinierungsreaktion mit einem Phosphonsäuredialkylester der allgemeinen Formel VI

$$(R^9O)_2\overset{O}{\overset{\|}{P}}-CR^3H-(CH_2)_m-Z \qquad (VI)$$

in dem $R^9$ ein $C_1$-$C_3$-Alkylrest ist, zu den erfindungsgemäßen Alkensäuresster der allgemeinen Formel I b;

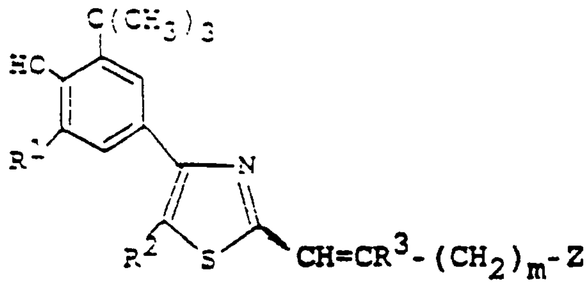

(I b)

in denen $R^1$, $R^2$, $R^3$, Z, m und $R^5$ die in Anspruch 1 erwähnte Bedeutung haben, umsetzt, oder
c) einen der oben beschriebenen Thiazolaldehyde der Formel V in einer Olefinierungsreaktion mit einem 3-Halogenpyrrolidinon der Formel VII

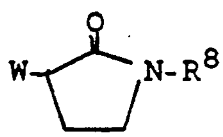

(VII)

gemeinsam mit einem Phosphin- oder Phosphorsäureester in Gegenwart einer starken Base zu den erfindungsgemäßen Verbindungen der Formel I c,

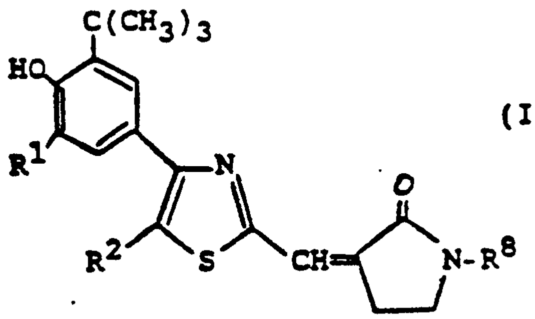

(I c)

umsetzt,
wobei in den Formeln W die bei Variante a) und $R^1$, $R^2$ und $R^8$ die in Anspruch 1 genannte Bedeutung besitzen, oder
d) eine Verbindung der Formel V mit einem Hydroxylamin der Formel VIII,

$$H_2N-O-(CH_2)_n-\overset{O}{\overset{\|}{C}}-X \qquad \text{(VIII)}$$

worin X und n die in Anspruch 1 genannte Bedeutung haben, zu den erfindungsgemäßen Verbindungen I d

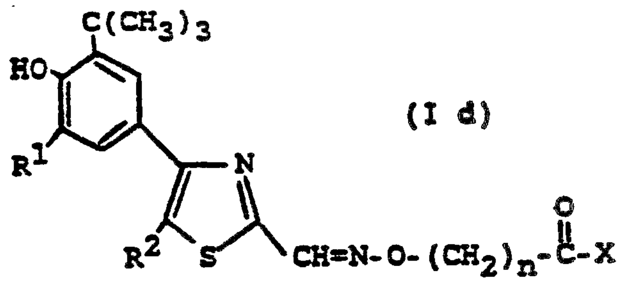

(I d)

kondensiert, oder

e) ausgehend von einem Hydroxymethylthiazol der allgemeinen Formel IX,

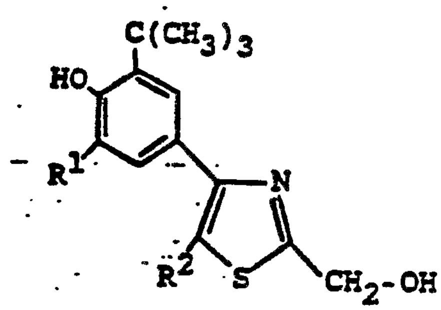

(IX)

mit einem Keton der Formel $R^3$-CO-$R^4$, wobei $R^1$, $R^2$ und $R^4$ die in Anspruch 1 angegebene Bedeutung haben, in Gegenwart eines tri- oder tetrahalogenierten Alkans und einer starken Base in die erfindungsgemäßen Verbindungen der Formel I e,

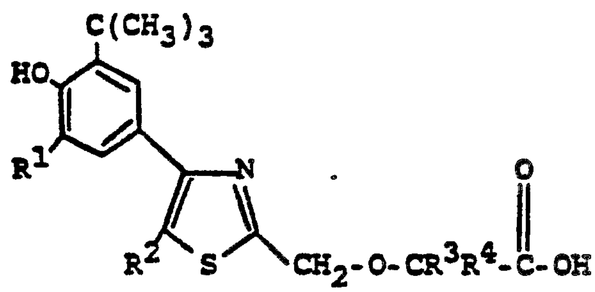

(I e)

umsetzt, oder

f) vorzugsweise Verbindungen der Formel Ia, Ib und Ic in der X = $R^5$O bedeutet, in Gegewart einer Base zu den Verbindungen der allgemeinen Formel I, in denen X = OH ist, verseift oder

g) Verbindungen der Formel I, in denen X = OH ist mit einem Alkohol $R^5$OH, Amin oder Hydroxylamin $R^6R^7$NH in Gegenwart eines Kondensationsmittels oder eines aktivierten Säurederivates in die Verbindung der allgemeinen Formel I überführt, in denen X für eine $R^5$O-oder $R^6R^7$N-Gruppe mit den für $R^5R^6$ und $R^7$ im Anspruch 1 genannten Bedeutung steht, oder

h) Verbindungen der Formel I, in der X einen $R^5$O-Rest darstellt, mit einer Base $R^6R^7$NH, in die Amide der allgemeinen Formel I, in denen X für eine $R^6R^7$N-Gruppe steht, umwandelt, oder

i) Verbindungen der Formel Ia, in denen $R^1$, $R^2$ und $R^5$, die im Anspruch 1 angegebene Bedeutung haben, $R^3$ Wasserstoff oder Methyl, $R^4$ = H und Y eine Einfachbindung bedeutet, durch katalytische Hydrierung der erfindungsgemäßen Verbindungen der allgemeinen Formel I b herstellt.

6. Verbindungen der Formel I sowie gegebenenfalls ihrer physiologisch verträglichen Salze gemäß einem oder mehreren der Ansprüche 1 - 4 oder wie erhalten nach dem Verfahren gemäß Anspruch 5 zur Verwendung als Heilmittel.

7. Arzneimittel, gekennzeichnet durch einen Gehalt an- oder bestehend aus- mindestens einer Verbindung

der Formel I und/oder mindestens einem ihrer physiologisch verträglichen Salze nach einem oder mehreren der Ansprüche 1 bis 4 und/oder mindestens einer nach dem Verfahren gemäß Anspruch 5 hergestellten Verbindung.

**8.** Arzneimittel nach Anspruch 7, dadurch gekennzeichnet, daß es zur Vorbeugung und Behandlung von Krankheiten bestimmt ist, bei denen die therapeutische Anwendung von Entzündungshemmern, Immunmodulatoren, Sauerstoffradikale desaktivierenden Mitteln und/oder Hemmstoffen des durch die 5-Lipoxygenase und/oder Cyclooxygenase vermittelten Arachidonsäureabbaus indiziert ist.

**9.** Arzneimittel nach Anspruch 7 oder 8, dadurch gekennzeichnet, daß es zur Vorbeugung und Behandlung von entzündlichen, insbesondere von entzündlich rheumatischen Erkrankungen verwendet wird.

**10.** Verfahren zur Herstellung eines Arzneimittels gemäß einem oder mehreren der Ansprüche 7 - 9 dadurch gekennzeichnet, daß man mindestens eine Verbindung der Formel I und/oder mindestens ein physiologisch verträgliches Salz einer solchen Verbindung und/oder eine der nach dem Verfahren gemäß Anspruch 5 erhaltenen Verbindungen mit einem physiologisch annehmbaren Träger und gegebenenfalls weiteren Zusatz- und/oder Hilfsstoffen in eine geeignete Darreichungssform bringt.

**11.** Verwendung von Verbindungen der Formel I und/oder gegebenenfalls deren physiologisch verträglichen Salze zur Herstellung von Arzneimitteln gemäß den Ansprüchen 8 und 9.

Patentansprüche für folgende Vertragsstaaten: ES, GR

**1.** Verfahren zur Herstellung von 2-substituierten 4-(3-tert.butyl-4-hydroxyphenyl)-thiazole der Formel I, in der

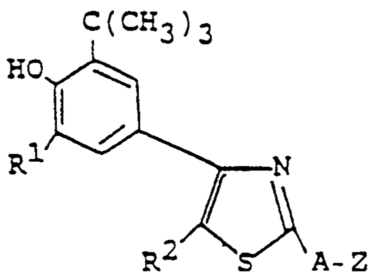

(I)

$R^1$
eine gesättigte oder ungesättigte, geradkettige oder verzweigte $C_1$-$C_5$-Alkylgruppe,
$R^2$
ein Wasserstoffatom oder ein Alkylrest mit 1 bis 3 C-Atomen,
A
eine Zwischenkette der Formel

$-(CH_2)_n-Y-CR^3R^4-$; $-CH=CR^3-(CH_2)_m-$ oder $-CH=N-O-(CH_2)_n-$

wobei Y eine Einfachbindung, ein Sauerstoff- oder Schwefelatom oder eine Carbonylgruppe,
$R^3$ und $R^4$ gleich oder verschieden sind und ein Wasserstoffatom oder einen Alkylrest mit bis zu 2 C-Atomen,
n eine Zahl von 1 bis 4 darstellen, und
m eine Zahl von 0 bis 3 und
Z
eine Tetrazol-, CN-Gruppe oder einen Rest der Formel

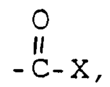

X

eine Hydroxygruppe oder ein Rest der allgemeinen Formel $R^5O$- oder $R^6R^7N$- , wobei $R^5$ ein geradkettiger oder verzweigter, gegebenfalls durch Hydroxy, $C_1$-$C_3$-Alkoxy oder $C_1$-$C_3$-Alkylamino-substituierter $C_1$-$C_4$-Alkylrest, $R^6$ und $R^7$ gleich oder verschieden sind und ein Wasserstoffatom, ein geradkettig oder verzweigter $C_1$-$C_6$-Alkylrest, oder für den Fall, daß $R^6$ ein Wasserstoffatom oder ein $C_1$-$C_4$-Alkylrest bedeutet, $R^7$eine Hydroxy-, $C_1$-$C_3$- Alkoxy- oder eine Tetrazol-5-yl-gruppe oder X mit den Strukturelementen -A-(C=O)- zusammen einen Rest der allgemeinen Formel II darstellt,

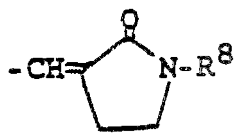

(II)

wobei $R^8$ ein Wasserstoffatom, einen $C_1$-$C_3$-Alkyl- oder $C_1$-$C_3$-Alkoxyrest bedeutet,
sowie physiologisch verträgliche Salze solcher Verbindungen der allgemeinen Formel I, in denen X eine Hydroxy- oder Hydroxyaminogruppe darstellt,
dadurch gekennzeichnet, daß man

a) ein Thiacarbonsäureamid der allgemeinen Formel III

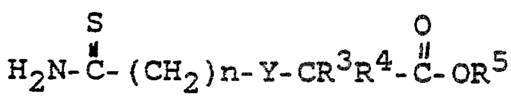

(III)

mit 2-Halogen-1-phenylalkanonen der Formel IV,

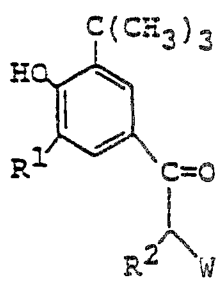

(IV)

in der $R^1$ und $R^2$ die in Anspruch 1 genannte Bedeutung haben und W für ein Halogen, vorzugsweise Chlor oder Brom steht, zu den erfindungsgemäßen Verbindungen der Formel Ia,

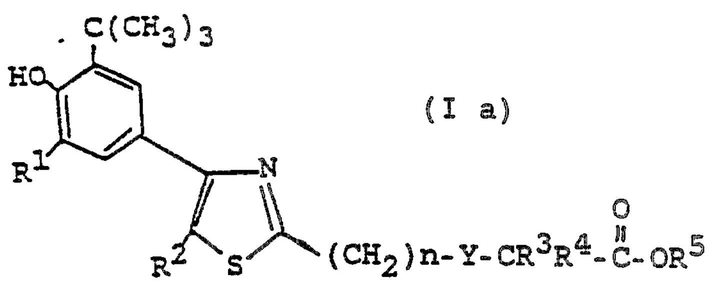

(I a)

mit den für $R^1$, $R^2$, $R^3$, $R^4$, $R^5$, Y und n in Anspruch 1 angegebenen Bedeutungen, umsetzt, oder

b) einen Thiazolaldehyd der allgemeinen Formel V,

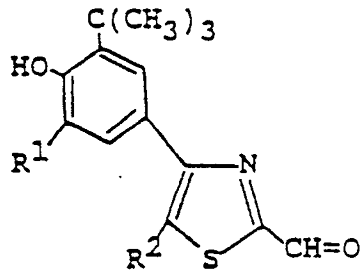

(V)

in einer Olefinierungsreaktion mit einem Phosphonsäuredialkylester der allgemeinen Formel VI

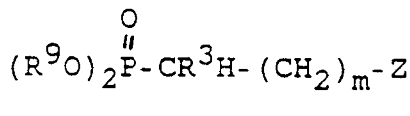

(VI)

in dem $R^9$ ein $C_1$-$C_3$-Alkylrest ist, zu den erfindungsgemäßen Alkensäureresster der allgemeinen Formel I b;

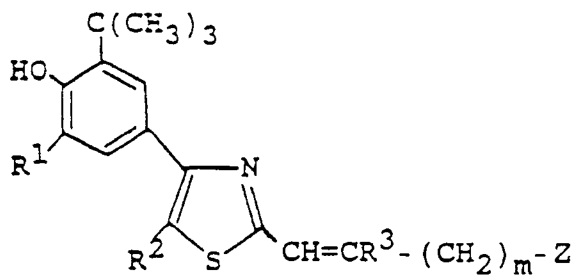

(I b)

in denen $R^1$, $R^2$, $R^3$,Z, m und $R^5$ die oben erwähnte Bedeutung haben, umsetzt, oder

c) einen der oben beschriebenen Thiazolaldehyde der Formel V in einer Olefinierungsreaktion mit einem 3-Halogenpyrrolidinon der Formel VII

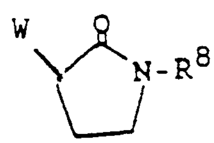

(VII)

gemeinsam mit einem Phosphin- oder Phosphorsäureester in Gegenwart einer starken Base zu den erfindungsgemäßen Verbindungen der Formel I c,

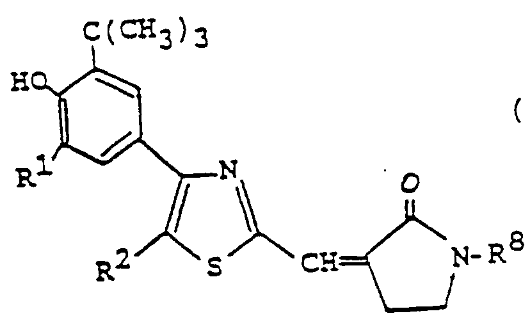

(I c)

umsetzt,

wobei in den Formeln W die bei Variante a) und $R^1$, $R^2$ und $R^8$ die oben genannte Bedeutung besitzen, oder

d) eine Verbindung der Formel V mit einem Hydroxylamin der Formel VIII, in dem

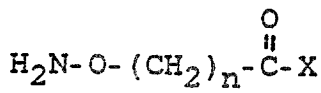
(VIII)

X und n die in Anspruch 1 genannte Bedeutung haben, zu den erfindungsgemäßen Verbindungen I d

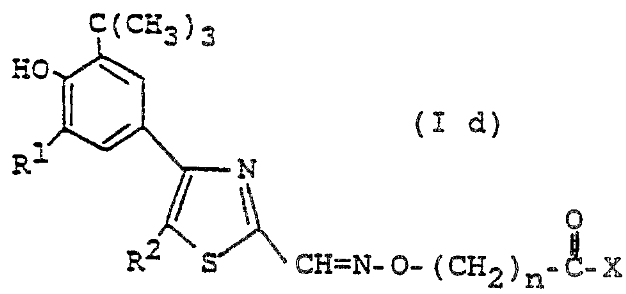
(I d)

kondensiert, oder

e) ausgehend von einem Hydroxymethylthiazol der allgemeinen Formel IX,

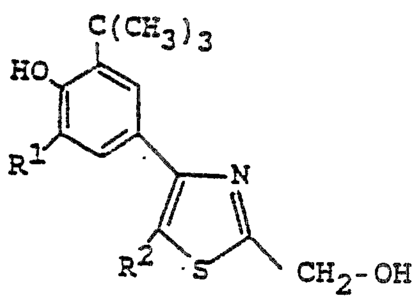
(IX)

mit einem Keton der Formel $R^3$-CO-$R^4$, wobei $R^1$, $R^2$ und $R^4$ die in Anspruch 1 angegebene Bedeutung haben, in Gegenwart eines tri- oder tetrahalogenierten Alkans und einer starken Base in die erfindungsgemäßen Verbindungen der Formel I e,

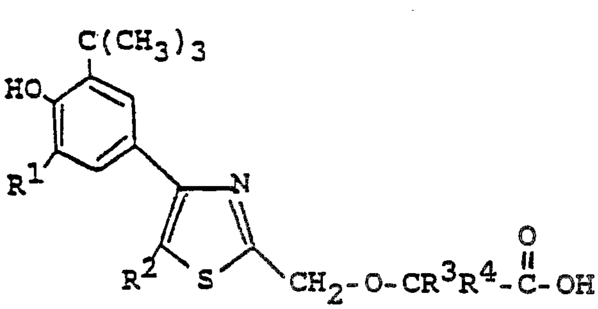
(I e)

umsetzt, oder

f) vorzugsweise Verbindungen der Formel Ia, Ib und Ic in der X = $R^5$O bedeutet, in Gegewart einer Base zu den Verbindungen der allgemeinen Formel I, in denen X = OH ist, verseift oder

g) Verbindungen der Formel I, in denen X = OH ist mit einem Alkohol $R^5$OH, Amin oder Hydroxylamin $R^6R^7$NH in Gegenwart eines Kondensationsmittels oder eines aktivierten Säurederivates in die Verbindung der allgemeinen Formel I überführt, in denen X für eine $R^5$O- oder $R^6R^7$N-

Gruppe mit den für $R^5R^6$ und $R^7$ im Anspruch 1 genannten Bedeutung steht, oder
h) Verbindungn der Formel I, in der X ein $R^5O$-Rest darstellt, mit einer Base $R^6R^7NH$, in die Amide der allgemeinen Formel I, in denen X für eine $R^6R^7N$-Gruppe steht, umwandelt, oder
i) Verbindungen der Formel Ia, in denen $R^1$, $R^2$ und $R^5$, die im Anspruch 1 angegebene Bedeutung haben, $R^3$ ein Wasserstoff oder Methyl, $R^4$ = H und Y eine Einfachbindung bedeutet, durch katalytische Hydrierung der erfindungsgemäßen Verbindungen der allgemeinen Formel I b erhält.

**2.** Verfahren nach Anspruch 1, dadurch gekennzeichnet, daß man eine Verbindung der allgemeinen Formel I,
in der
$R^1$
eine tert.Butyl- oder Methylgruppe darstellt,
$R^2$
für Wasserstoff oder Methyl,
A
für eine Zwischenkette der Formel

$-(CH_2)_n-Y-CR^3R^4-$

steht, wobei Y eine Einfachbindung oder ein Sauerstoffatom, $R^3$ und $R^4$ gleich oder verschieden sind und ein Wasserstoffatom oder einen Methylrest, und n eine Zahl von 1 oder 2 darstellen, und
X
eine Hydroxygruppe oder ein Rest der allgemeinen Formel $R^5O$-, oder $R^6R^7N$- darstellt, wobei $R^5$ ein geradkettiger oder verzweigter $C_1$-$C_3$-Alkylrest, $R^6$ ein Wasserstoffatom oder ein Methylrest und $R^7$ eine Hydroxy-, Methoxy- oder Tetrazol-5-yl-gruppe bedeutet, herstellt.

**3.** Verfahren nach Anspruch 1, dadurch gekennzeichnet, daß man eine Verbindung der allgemeinen Formel I,
in der
$R^1$
für eine tert.Butylgruppe
$R^2$
für Wasserstoff oder Methyl,
A
für eine Zwischenkette der Formel

$-(CH_2)_n-Y-CR^3R^4-$

steht, wobei Y eine Einfachbindung, $R^3$ und $R^4$ ein Wasserstoffatom und n eine Zahl von 1 oder 2 darstellen und
X
eine Hydroxygruppe oder $R^6R^7N$-Rest darstellt, wobei $R^6$ ein Wasserstoffatom oder ein Methylrest und $R^7$ eine Hydroxy-, oder Methoxygruppe bedeutet, herstellt.

**4.** Verfahren nach Anspruch 1, dadurch gekennzeichnet, daß man 3-[4-(3,5-Di-tert.butyl-4-hydroxyphenyl)-thiazol-2-yl]-propionsäure herstellt.

**5.** Verwendung von Verbindungen der Formel I und/oder gegebenenfalls deren physiologisch verträglichen Salze zur Herstellung von Arzneimitteln zur Vorbeugung und Behandlung von Krankheiten, bei denen die therapeutische Anwendung von Entzündungshemmern, Immunmodulatoren, Sauerstoffradikale desaktivierenden Mitteln und/oder Hemmstoffen des durch die 5-Lipoxygenase und oder Cyclooxygenase vermittelten Arachidonsäureabbaus indiziert ist.

**6.** Verwendung von Verbindungen der Formel I nach Anspruch 5, dadurch gekennzeichnet, daß sie zur Vorbeugung und Behandlung von entzündlichen, insbesondere von entzündlich rheumatischen Erkrankungen verwendet werden.

**7.** Verfahren zur Herstellung eines Arzneimittels gemäß den Ansprüche 5 oder 6, dadurch gekennzeichnet, daß man mindestens eine Verbindung der Formel I und/oder mindestens ein physiologisch verträgliches Salz einer solchen Verbindung mit einem physiologisch annehmbaren Träger und gegebenenfalls weiteren Zusatz- und/oder Hilfsstoffen in eine geeignete Darreichungsform bringt.